# EUROPÄISCHE PATENTSCHRIFT

(11) **EP 2 764 078 B1**
(45) Veröffentlichungstag und Bekanntmachung des Hinweises auf die Patenterteilung: **09.08.2017**
(21) Anmeldenummer: 12753944.3
(22) Anmeldetag: 28.08.2012
(51) Int. Cl.: C11D 3/32, C11D 17/00, C07C 235/74, C11D 3/20

(54) **HYDROGELBILDNER ENTHALTENDE WASCH- ODER REINIGUNGSMITTEL**
WASHING OR CLEANING AGENT COMPRISING A HYDROGEL FORMER
DETERGENTS OU NETTOYANTS CONTENANT DES AGENTS DE FORMATION D'HYDROGEL

(30) Priorität: 04.10.2011 DE 102011083942
(43) Veröffentlichungstag der Anmeldung: 13.08.2014
(73) Patentinhaber: Henkel AG & Co. KGaA, 40589 Düsseldorf (DE)
(72) Erfinder: KROPF, Christian, 40724 Hilden (DE); SUNDER, Matthias, 40593 Düsseldorf (DE); SCHMIEDEL, Peter, 40591 Düsseldorf (DE); PEGELOW, Ulrich, 40597 Düsseldorf (DE); TILLER, Jörg C., 58313 Herdecke (DE); MÜLLER, Marco, 79312 Emmendingen (DE); TACHIKAWA, Kaoru, 44137 Dortmund (DE); WILLEMSEN, Yvonne, 41352 Korschenbroich (DE)
(86) Internationale Anmeldenummer: PCT/EP2012/066644
(87) Internationale Veröffentlichungsnummer: WO 2013/050201

(56) Entgegenhaltungen:
- EP-A1- 1 318 191
- EP-A1- 2 365 052
- DE-A1- 19 933 404
- LOOS DE M ET AL: "DESIGN AND APPLICATIONS OF SELF-ASSEMBLED LOW MOLECULAR WEIGHT HYDROGELS", EUROPEAN JOURNAL OF ORGANIC CHEMISTRY, WILEY - V C H VERLAG GMBH & CO. KGAA, DE, Nr. 17, 1. September 2005 (2005-09-01), Seiten 3615-3631, XP002499285, ISSN: 1434-193X, DOI: 10.1002/EJOC.200400723 in der Anmeldung erwähnt
- ESTROFF L A ET AL: "WATER GELATION BY SMALL ORGANIC MOLECULES", CHEMICAL REVIEWS, AMERICAN CHEMICAL SOCIETY, US, Bd. 104, Nr. 3, 1. Januar 2004 (2004-01-01), Seiten 1201-1218, XP003016413, ISSN: 0009-2665, DOI: 10.1021/CR0302049 in der Anmeldung erwähnt

## Beschreibung

Die Erfindung liegt auf dem Gebiet der Wasch- oder Reinigungsmittel und betrifft Wasch- oder Reinigungsmittel, die Hydrogelbildner enthalten.

Als Hydrogele werden im Stand der Technik im allgemeinen Wasser enthaltende Gele auf der Basis hydrophiler, aber wasserunlöslicher Polymerer, die als dreidimensionale Netzwerke vorliegen, bezeichnet. Diese Netzwerke quellen in Wasser unter weitgehender Formerhaltung auf. Die Netzwerkbildung erfolgt bei Einsatz polymerer Hydrogelbildner größtenteils über chemische Verknüpfung der einzelnen Polymerketten. Beispiele wasserunlöslicher Polymere, welche als Hydrogelbildner eingesetzt werden, basieren z.B. auf Poly(meth)acrylsäuren, Poly(meth)acrylaten, Polyvinylpyrrolidon, Polyvinylalkohol oder Pektin.

Entsprechend ist die Erzeugung von Hydrogelen unter Einsatz polymerer Hydrogelbildner auf Oberflächen bekannt, um diese in ihren Eigenschaften zu modifizieren. Mit Verfahren wie der Layer-by-Layer-Technik können dabei auch dickere Schichten erzeugt werden. Allerdings erfordert die Layer-by-Layer-Technik die Verwendung von mindestens zwei unterschiedlichen Polymeren und ein mehrstufiges Verfahren zur Erzeugung der Schichten. Der damit einhergehende erhöhte Aufwand wird als nachteilig empfunden.

Ebenso nachteilig ist beim Einsatz wasserunlöslicher Polymere als Hydrogelbildner grundsätzlich das Zurückbleiben der Polymere beim Auflösen der Hydrogelschichten, z.B. durch große Verdünnung. Die betreffenden Polymere bilden somit einen Ballast. Dieser Ballast ist insbesondere bei Einsatz in Wasch- oder Reinigungsmitteln unerwünscht, da er z.B. zu unerwünschten Rückständen bei der Anwendung und damit z.B. zu einer funktionellen oder ästhetischen Beeinträchtigung führen kann. Ebenso ist es möglich, dass die betreffenden Polymere nur schwer abbaubar sind, was dem Grundbedürfnis nach einer größtmöglichen Minimierung ökologischer Belastungen entgegensteht.

Die Aufgabe der vorliegenden Erfindung lag vor diesem Hintergrund darin, die genannten Nachteile zu überwinden.

Überraschend wurde nun im Rahmen der vorliegenden Erfindung gefunden, dass die Verwendung von bestimmten Hydrogelbildnern in Wasch- oder Reinigungsmitteln sehr gut zur Erzeugung von Hydrogelschichten auf harten oder textilen Oberflächen einsetzbar ist. Der Einsatz von mindestens zwei unterschiedlichen niedermolekularen Hydrogelbildnern und ein mehrstufiges Verfahren sind dabei zur Erzeugung der Schichten nicht notwendig. Weiterhin resultiert kein unlöslicher Polymerballast.

Solches ist aus dem Stand der Technik nicht bekannt. Es wir dort in US 6,576,679 B2 die Bildung eines Hydrogels unter Verwendung eines kationischen niedermolekularen amphiphilen Gelbildners vom Glutaminderivat-Typ in Gegenwart bestimmter Anionen beschrieben. Weiterhin wird in EP 1 553 109 A1 ein molekular-orientiertes Polymergel beschrieben, das erhalten wird durch Selbstanordnung einer selbstorganisierenden amphiphilen Verbindung und eines Monomers, das mit der amphiphilen Verbindung wechselwirkt und nachfolgendes Polymerisieren des Monomers. In WO 03/097587 ist eine Substanzklasse niedermolekularer Hydrogelbildner beschrieben, die sich für die Verdickung bzw. Gelierung von Lösungsmitteln wie z.B. Wasser eignen. Die Erzeugung von Hydrogelschichten auf Oberflächen aus wässriger Lösung heraus wird nicht beschrieben. Weiterhin werden in den beiden nachfolgenden Übersichtsartikel zahlreiche niedermolekulare Hydrogelbildner genannt: (a) Loos, Feringa, Esch, Eur. J. Org. Chem. 2005, 3615, (b) Estroff, Hamilton, Chem. Rev. 2004, 104, 1201. Der Einsatz entsprechender Hydrogelbildner in Wasch- oder Reinigungsmitteln zur Erzeugung von Hydrogelschichten auf Oberflächen aus wässriger Lösung heraus wird jedoch nicht beschrieben.

EP 2 365 052 A1 offenbart niedermolekulare Hydrogelbildner enthaltend Amidogruppen zum Einsatz in Wasch- und Reinigungsmitteln.

EP 1 318 191 A1 beschreibt niedermolekulare Haftvermittler in Reinigungsmitteln mit einer Ausprägung zur Gelbildung.

DE 199 33 404 A1 offenbart Reinigungsmittel enthaltend Zuckertenside zur Erhöhung der Viskosität.

Ein erster Gegenstand der Erfindung ist vor diesem Hintergrund ein Wasch- oder Reinigungsmittel, welches zumindest einen Hydrogelbildner mit einem Molekulargewicht kleiner als 3500 g/Mol umfasst,
wobei der Hydrogelbildner der folgenden Formel (I) genügt: 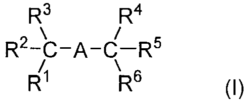 wobei den Resten A, R¹ bis R⁶ folgende Bedeutung zukommt:
A = -(CH₂)ₙ- mit n = 1 bis 24, vorzugsweise 6 bis 18, insbesondere 8 bis 14,
R¹ bis R⁶, jeweils unabhängig voneinander: Alkyl-, Hydroxyalkyl, Aminoalkyl-, -COOR, -CONRR oder 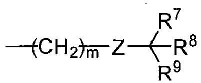 mit m = 0 - 10
   darin ist Z = -CONH-(CH₂)ᵣ-, -COO-(CH₂)ᵣ-, -O-(Ch₂)ᵣ- oder -CONR-(CH₂)ᵣ-,
   mit r = 0 bis 10
   und R⁷ bis R⁹ unabhängig voneinander = H, Alkyl, Hydroxyalkyl, Aminoalkyl, -CONRR, -COOR oder Monosaccharide,
   wobei R = H, Alkyl oder Aryl ist,
   wobei der Hydrogelbildner bei Konzentrationen unterhalb der minimalen Gelbildungskonzentration wasserlöslich ist,
   wobei die minimale Gelbildungskonzentration diejenige Konzentration ist, bei welcher sich in einer binären Lösung aus Wasser und Hydrogelbildner bei T = 25°C erstmals eine Fließgrenze von zumindest 0,05 Pa ausbildet,
   und wobei diese minimale Gelbildungskonzentration unterhalb 2 Gew.-% Hydrogelbildner liegt, bezogen auf das System bestehend aus Wasser und Hydrogelbildner,
   und wobei das Wasch- oder Reinigungsmittel 0,1 bis 40 Gew.-% Tensid(e) umfasst.

Die Fließgrenzen werden gemessen mit einem Rotationsrheometer der Firma TA-Instruments, Typ AR G2. Hierbei handelt es sich um ein sogenanntes schubspannungskontrolliertes Rheometer. Zur Messung einer Fließgrenze mit einem schubspannungskontrollierten Rheometer sind in der Literatur verschiedene Verfahren beschrieben, die dem Fachmann bekannt sind.

Zur Bestimmung der Fließgrenzen im Rahmen der vorliegenden Erfindung wurde folgendermaßen vorgegangen:
Die Proben wurden im Rheometer mit einer mit der Zeit ansteigenden Schubspannung s(t) beaufschlagt. Beispielsweise kann die Schubspannung im Laufe von 10 Minuten vom kleinstmöglichen Wert (z.B. 2 mPa) auf z.B. 10 Pa gesteigert werden. Als Funktion dieser Schubspannung wird die Deformation g der Probe gemessen. Die Deformation wird in einem doppellogarithmischen Plot gegen die Schubspannung aufgetragen. Sofern die untersuchte Probe eine Fließgrenze aufweist, kann man in diesem Plot deutlich zwei Bereiche unterscheiden. Unterhalb einer gewissen Schubspannung findet man eine rein elastische Deformation. Die Steigung der Kurve g(s) (log-log-Plot) in diesem Bereich ist eins. Oberhalb dieser Schubspannung beginnt der Fließbereich und die Steigung der Kurve ist sprunghaft höher. Diejenige Schubspannung bei der das Abknicken der Kurve erfolgt, also der Übergang von der elastischen in eine plastische Deformation, markiert die Fließgrenze. Eine bequeme Bestimmung des Knickpunktes ist durch Anlegen von Tangenten an die beiden Kurventeile möglich. Proben ohne Fließgrenze weisen keinen charakteristischen Knick in der Funktion g(s) auf.

Ein Hydrogelbildner im Sinne der Erfindung hat das Vermögen zur Selbstorganisation unter Hydrogelbildung. Gele als solche müssen zumindest zwei Komponenten umfassen, nämlich eine Flüssigkeit und einen festen Bestandteil, der ein dreidimensionales Netzwerk bildet, welches in seinem Innern die Flüssigkeit einschließt. In Hydrogelen im Sinne der Erfindung bildet der feste Bestandteil ein dreidimensionales Netzwerk, welches in seinem Innern Wasser oder eine wässrige Lösung einschließt.

Ein Hydrogelbildner im Sinne der Erfindung ist dementsprechend eine Verbindung, die das Vermögen zur Selbstorganisation unter Hydrogelbildung besitzt.

Bei den erfindungsgemäßen Hydrogelen handelt es sich nicht um Hauptvalenzgele, d.h. der Gerüstaufbau im Netzwerk ist das Resultat intermolekularer bzw. physikalischer Wechselwirkungen (z.B. Dipol-Dipol-Kräfte, Wasserstoffbrückenbindungen, aromatische π-π-Wechselwirkungen, Vander-Waals-Wechselwirkungen, ionische Wechselwirkung, Ion-Dipol-Wechselwirkung, Kation-π-Wechselwirkung oder Coulomb-Kräfte) zwischen den Molekülen und nicht das Resultat kovalenter Bindungen zwischen den Bausteinen bzw. Molekülen. Die dreidimensionale Netzwerkstruktur kann sich auch durch unspezifische Verschlaufungen oder durch Kontakt zwischen den Bausteinen ergeben, wobei diese Bausteine dann Aggregate kleinerer Moleküle sind, welche ihrerseits durch nicht-kovalente Bindungen z.B. zu Stäbchen oder Ketten verknüpft sind. Die Bildung der erfindungsgemäßen Hydrogele beruht also auf supramolekularen Wechselwirkungen der erfindungsgemäßen Hydrogelbildner. So entstehen durch Selbstanordnung Hydrogelschichten.

Die erfindungsgemäße Bildung der Hydrogele auf den Oberflächen beruht zusätzlich auf Wechselwirkungen der erfindungsgemäßen Hydrogelbildner mit der zu belegenden Oberfläche. Dadurch entstehen auf den Oberflächen Gelschichten bereits bei Konzentrationen, bei denen in der Lösung noch keine Gelierung erfolgt (d.h. die minimale Gelbildungskonzentration in Gegenwart der Oberfläche ist kleiner als die minimale Gelbildungskonzentration in der Lösung), was ein großer Vorteil ist. Dabei ist die minimale Gelbildungskonzentration die Konzentration eines Hydrogelbildners in homogener wässriger Lösung, bei bzw. oberhalb der er ein Hydrogel ausbildet, d.h. durch einen Selbstorganisationsprozess ein Netzwerk entsteht, in welchem Wasser eingeschlossen wird, wodurch die Flüssigkeit in ein kohärentes Gel überführt wird. Die Hydrogelschichten sind also erzeugbar, ohne dass die wässrige Lösung selbst vergelt bzw. verdickt werden muss.

Niedermolekulare Hydrogelbildner sind, wie zuvor erwähnt, an sich aus der Literatur bekannt. Verwiesen sei hier insbesondere auf die bereits erwähnten Übersichtsartikel und die darin genannten Hydrogelbildner: (a) Loos, Feringa, Esch, Eur. J. Org. Chem. 2005, 3615, (b) Estroff, Hamilton, Chem. Rev. 2004, 104, 1201. Prinzipiell können alle literaturbekannten Hydrogelbildner und insbesondere die in den beiden erwähnten Übersichtsartikeln genannten Hydrogelbildner im Sinne der Erfindung eingesetzt werden, sofern sie bei Konzentrationen unterhalb der minimalen Gelbildungskonzentration wasserlöslich sind,
wobei die minimale Gelbildungskonzentration diejenige Konzentration ist, bei welcher sich in einer binären Lösung aus Wasser und Hydrogelbildner bei T = 25°C erstmals eine Fließgrenze von zumindest 0,05 Pa ausbildet,
und wobei diese minimale Gelbildungskonzentration unterhalb 2 Gew.-% Hydrogelbildner liegt, bezogen auf das System bestehend aus Wasser und Hydrogelbildner, und sofern sie ein Molekulargewicht kleiner als 3500 g/Mol aufweisen.

Es entspricht einer bevorzugten Ausführungsform der Erfindung wenn der erfindungsgemäße Hydrogelbildner ein Molekulargewicht kleiner 3000 g/mol, vorzugsweise kleiner 2500 g/mol, in weiter vorteilhafter Weise kleiner 2000 g/mol, insbesondere kleiner 1500 g/mol aufweist.

Viele der bekannten niedermolekularen Hydrogelbildner lassen sich als Amphiphile klassifizieren. Amphiphile umfassen einen polaren (hydrophilen) und einen apolaren (hydrophoben) Teil.

Ganz besonders bevorzugte erfindungsgemäß einsetzbare Hydrogelbildner genügen der folgenden Formel (II): 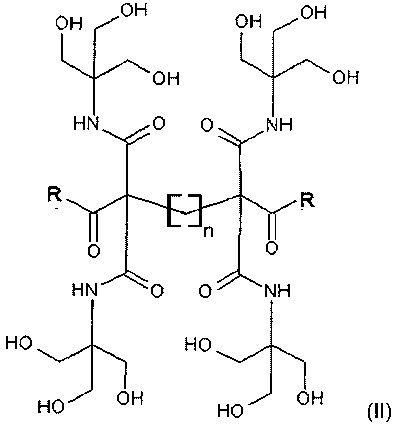
mit n = 1 bis 24, vorzugsweise 6 bis 18, insbesondere 8 bis 14, z.B. 12,
und R= HO-, Alkyl-O- (wie insbesondere C₂H₅-O-) oder (CH₂OH)₃C-NH-.

Wird in dem erfindungsgemäßen Wasch- oder Reinigungsmittel zumindest ein Hydrogelbildner eingesetzt, welcher der Formel (II) genügt, so liegt eine ganz besonders bevorzugte Ausführungsform der Erfindung vor.

Im Rahmen der vorliegenden Erfindung bevorzugt zusätzlich einsetzbare Hydrogelbildner genügen folgender grundsätzlicher Formel (I) 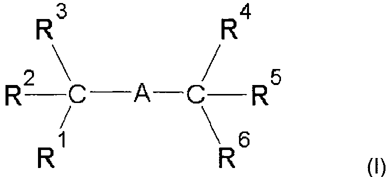 wobei den Resten A, R¹ bis R⁶ dabei folgende Bedeutung zukommt: A= 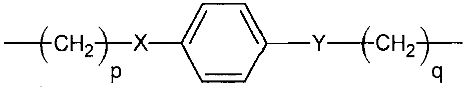 mit p, q unabhängig voneinander = 0 bis 20, z.B. 1 bis 16;
mit X, Y = 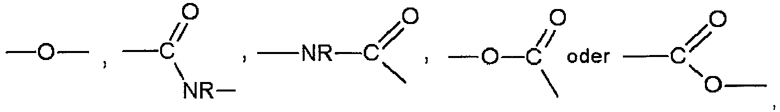 wobei X und Y unabhängig voneinander gewählt werden können, und R = H, Alkyl oder Aryl ist, R¹ bis R⁶, jeweils unabhängig voneinander: Alkyl- , Hydroxyalkyl-, Aminoalkyl-, -COOR, -CONRR oder 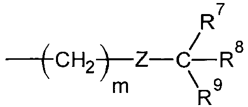 m=0-10
darin ist Z = -CONH-(CH₂)ᵣ-, -COO-(CH₂)ᵣ-, -O-(CH₂)ᵣ- oder -CONR-(CH₂)ᵣ-,
mit r= 0 bis 10
und R⁷ bis R⁹ unabhängig voneinander = H, Alkyl, Hydroxyalkyl, Aminoalkyl, -CONRR, -COOR oder Monosaccharide,
wobei R = H, Alkyl oder Aryl.

Erfindungsgemäß ganz besonders bevorzugt zusätzlich einsetzbare Hydrogelbildner genügen einer der folgenden Formeln (III), (IV) oder (V): 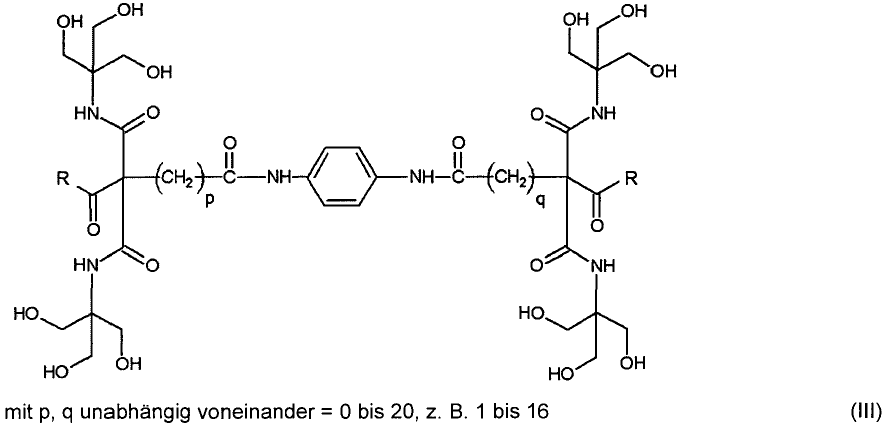 und R = HO-, Alkyl-O- (wie insbesondere C₂H₅-O-) oder (CH₂OH)₃C-NH-. 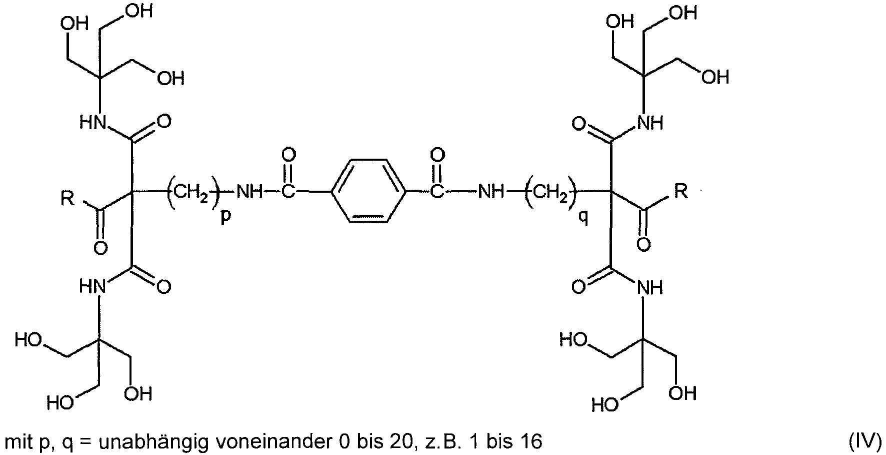 und R = HO-, Alkyl-O- (wie insbesondere C₂H₅-O-) oder (CH₂OH)₃C-NH-.

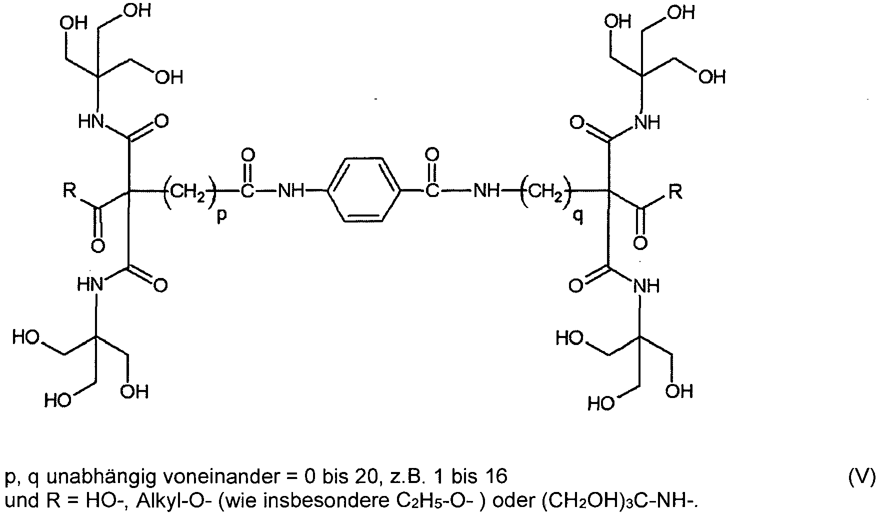

Weitere im Rahmen der vorliegenden Erfindung besonders bevorzugt zusätzlich einsetzbare Hydrogelbildner genügen vorzugsweise folgender grundsätzlicher Formel (VI): 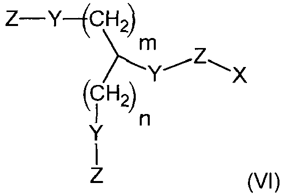
mit n = 0 bis 10, m = 0 bis 24
Y = 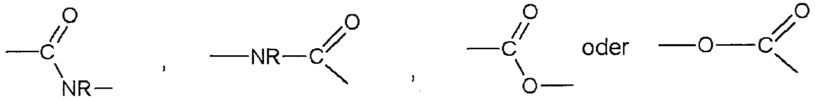
X = H, Alkyl, Aryl, Alkylarylether, 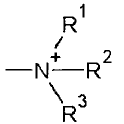 oder 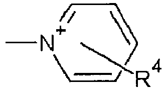 mit Halogenid, Alkylsulfonat, Arylsulfonat als Gegenion im Falle einer kationischen Verbindung
Z = H, Alkyl, Aryl oder Alkylarylether
R¹ bis R⁴ unabhängig voneinander = H, Alkyl, Hydroxyalkyl oder Aryl
R = H, Alkyl oder Aryl.

Besonders bevorzugte Vertreter dieser Substanzklasse gemäß der grundsätzlichen Formel (VI) sind die folgenden: 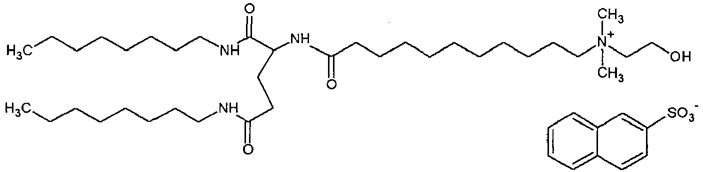 sowie

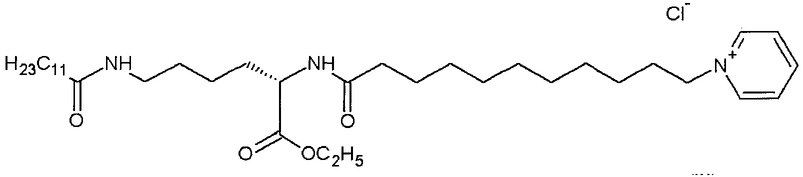

Dabei können als Anionen auch andere geeignete fungieren.

Der erfindungsgemäße Gegenstand ermöglicht die Erzeugung von Hydrogelschichten auf harten oder textilen Oberflächen (vorzugsweise durch die Oberflächen induziert) auch in Gegenwart von Tensiden aus wässriger Lösung heraus, insbesondere im Zusammenhang mit maschinellen Wasch- oder Reinigungsprozessen.

Der erfindungsgemäße Gegenstand geht mit einer Reihe von Vorteilen einher. Er ermöglicht z.B. die Hydrophilierung der Oberflächen. Dies ermöglicht eine erleichterte Entfernbarkeit bzw. Auswaschbarkeit von Schmutz von diesen Oberflächen (sogenannter "soil-release"-Effekt). Dies ermöglicht ferner ein verbessertes Schmutzabweisevermögen der Oberflächen, so dass bereits das Anschmutzen der Oberfläche erschwert ist (sogenannter "soil-repel"-Effekt).

Ferner wird bei Textilien der Griff der gewaschenen Produkte positiv beeinflusst. Insbesondere baumwollhaltige Textilien zeigen nach dem Waschen oft einen harten Griff. Mit Hilfe des erfindungsgemäßen Wasch- oder Reinigungsmittels wird dagegen ein Weichgriff der behandelten Textilien erzeugt.

Ein weiterer Vorteil liegt darin, dass bei der Schichtbildung, d.h. bei der Erzeugung von Hydrogelschichten auf harten oder weichen (insbesondere textilen) Oberflächen, Wirkstoffe in die Schichten ein- bzw. an die Schichten angelagert werden können, so dass die Schichten als Depot bzw. Reservoir für Wirkstoffe (wie z.B. Parfüms, Pflegestoffe, antimikrobielle Stoffe) dienen können. Die Wirkstoffe können dabei in der im Gel eingeschlossenen Flüssigkeit gelöst sein, sie können im Inneren der Strukturen eingeschlossen sein und/oder auf ihnen adsorbiert sein. Sie können sogar Bestandteil der die Gelschicht aufbauenden Strukturen sein.

Die erfindungsgemäßen Wasch- oder Reinigungsmittel haben somit den Vorteil, dass sie eine retardierte Freisetzung von Wirkstoffen, insbesondere Riechstoffen und antimikrobiellen Stoffen, welche in den Gelschichten gespeichert sind, ermöglichen. Bei der Anwendung des Wasch- oder Reinigungsmittels, z.B. bei der Textilwäsche kommt es zur Bildung von Hydrogelschichten auf dem zu reinigenden Gut. Auf diese Weise wird eine gezielte Wirkstofffreisetzung direkt auf dem behandelten Gut ermöglicht, so dass das Leistungsprofil des gesamten Mittels erhöht wird. Dabei kommt insbesondere der Duftwirkung eine besondere Bedeutung zu, da die Produktleistung in vielen Fällen vom Verbraucher proportional zum Wohlgeruch beurteilt wird. Die Freisetzung der Aktivstoffe, insbesondere Duftstoffe aus der Gelschicht, kann z.B. auf diffusivem Weg erfolgen, bei dem die Aktivstoffe, insbesondere Duftstoffe, langsam und kontinuierlich freigesetzt werden. Das vorliegende Wasch- oder Reinigungsmittel ermöglicht somit eine lang anhaltende und kontinuierliche Aktivstofffreisetzung, insbesondere eine lang anhaltende Beduftung der zu reinigenden Güter sowie eine gezielte Aktivstofffreisetzung direkt auf dem Zielobjekt. Ein besonderer Vorteil der erfindungsgemäßen Hydrogelschicht liegt darin, dass bei Auflösung der Hydrogelschichten diese wieder in ihre niedermolekularen Bestandteile zerfallen, so dass z.B. keine schwer abbaubaren Polymere zurückbleiben.

Nachfolgend werden noch weitere besonders vorteilhafte, im Sinne dieser Erfindung einsetzbare Hydrogelbildner genannt:
a) N,N'-bis(alkylamino)oxalamide,
   wie insbesondere 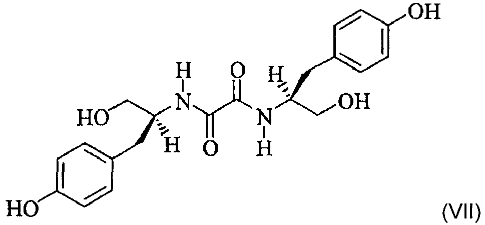
b) Bis-Harnstoff Dicarbonsäure basierte Hydrogelbildner, wie insbesondere 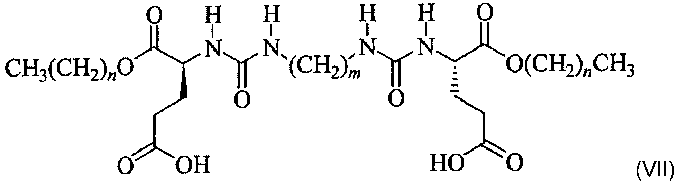 mit n = 6 bis 18 und m = 1 bis 18, z.B. mit n = 11 und m = 8.
c) Lysin basierte Bis-Amid -Carboxylat, -Pyridinium oder -Ammonium Verbindungen, wie insbesondere 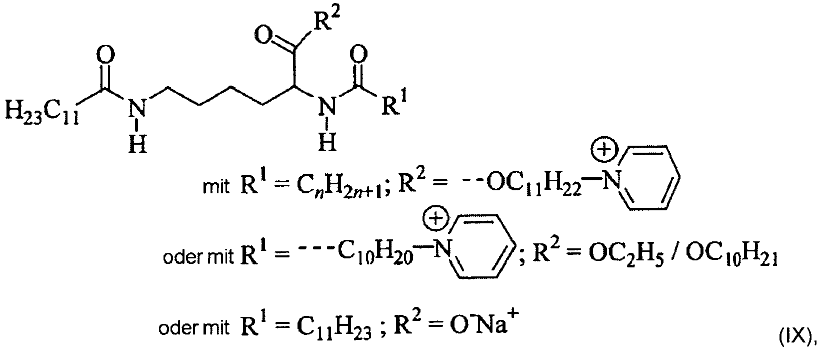 mit n = 1 bis 20
   oder wie insbesondere 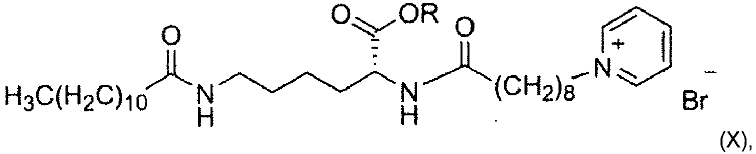 mit R = Me oder Et
   oder wie insbesondere 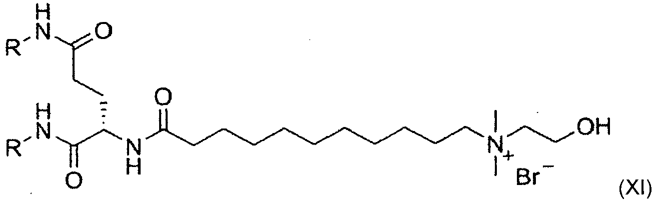 mit 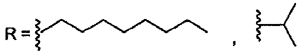 oder 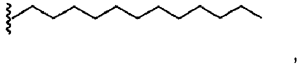
d) Cyclohexan-1,2-Bis-Harnstoff Amide,
   wie insbesondere 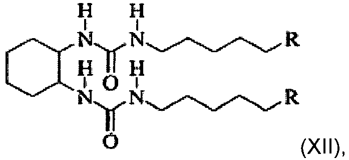 mit R = OH, CH₂OH oder CH₂NH₂
e) cis-cis-1,3,5-Cyclohexan tris Aminosäureamide,
   wie insbesondere 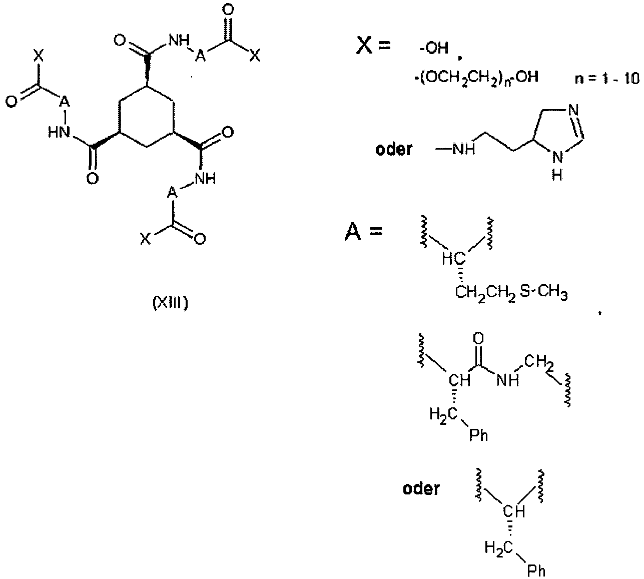
f) N-Alkylaldonamide,
   wie insbesondere N-Dodecylgluconamid 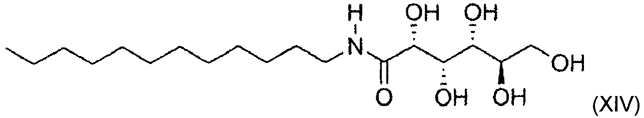
g) Aminosäure basierte bzw. Serin basierte Hydrogelbildner
   wie insbesondere N-Dodecanoyl-(D- und L-)Serin 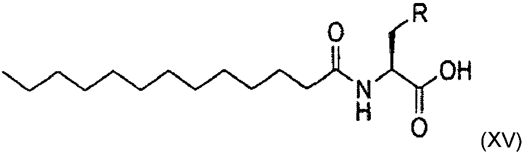 mit R = -H, -OH, -CH₂-COOH oder -(CH₂)₂-COOH
   oder insbesondere 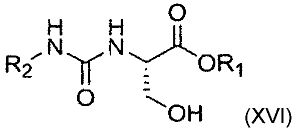
   mit R₁ = H, Alkyl oder Aryl
   und mit R₂ = Alkyl
h) Bola-Amphiphile (das ist die Sammelbezeichnung für Amphiphile, deren hydrophobe Gruppe, im allgemeinen Kohlenwasserstoff-Ketten, in α,ω-Stellung mit zwei (hydrophilen, ionischen und/oder nichtionischen) polaren Gruppen substituiert ist),
   wie insbesondere Zucker-Derivate oder Aminosäure-basierte Bola-Amphiphile: 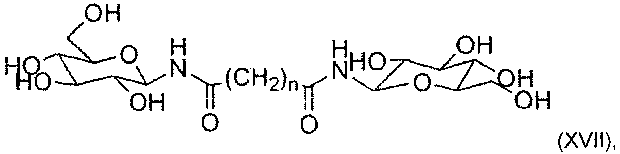 mit n = 6, 9, 10, 11, 12, 13 oder 14, oder 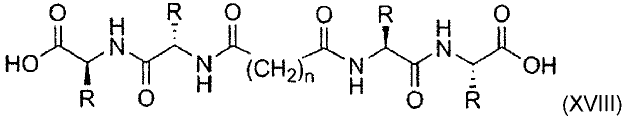
   mit n = 8 oder 10
   mit R = H, Alkyl oder Aryl oder 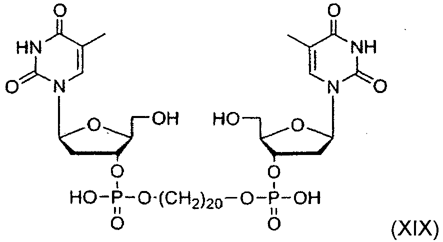 oder 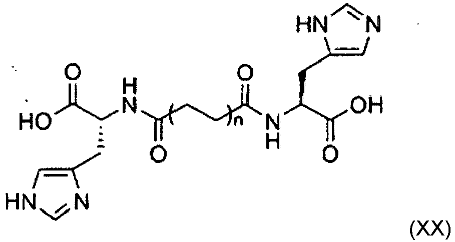 mit n = 5, 6 oder 10
i) Bis (Aminosäure)-oxalyl-Amid mit sperrigen (z.B. Phenyl oder Isopropyl)-Seitenketten,
   wie insbesondere 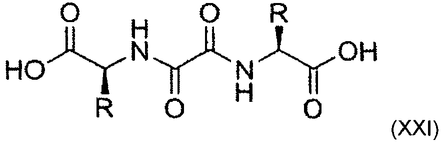 mit R = Alkyl oder Aryl
   z.B.

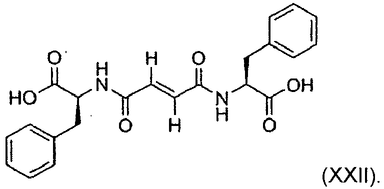

Gemäß einer bevorzugten Ausführungsform der Erfindung enthält das erfindungsgemäße Mittel eine Gesamtmenge an Hydrogelbildner von 0,01 bis 20 Gew.-%, vorzugsweise von 0,1 bis 5 Gew.-%, bezogen auf das gesamte Mittel. Insbesondere innerhalb der genannten Konzentrationsbereiche lassen sich bei der Anwendung der entsprechenden Wasch- oder Reinigungsmittel die gewünschten Hydrogelschichten auf harten oder weichen Oberflächen besonders gut erzeugen.

Das erfindungsgemäße Wasch- oder Reinigungsmittel enthält neben den beschriebenen Hydrogelbildnern vorzugsweise noch weitere Inhaltstoffe, nämlich insbesondere zumindest Tenside und/oder Gerüststoffe. Tenside sind in Mengen von 0,1 bis 40 Gew.-% enthalten.

Zunächst sei klargestellt, dass der Begriff der Waschmittel im Sinne dieser Erfindung insbesondere Textilwaschmittel als auch Textilnachbehandlungsmittel (wie vorzugsweise Weichspüler, Duftspüler, Konditioniertücher für die Anwendung im Wäschetrockner, Hygienespüler usw.) umfasst. Textilwaschmittel ist die Bezeichnung für die beim Waschen von Textilerzeugnissen benötigten, z.B. in Form von Pulvern, Granulaten, Perlen, Tabletten, Pasten, Gelen, Tüchern, Stücken oder Flüssigkeiten vorliegenden Formulierungen, die vorzugsweise in wässrigen Lösungen insbesondere in Waschmaschinen eingesetzt werden. Weichspüler sind Textilnachbehandlungsmittel zur Pflege von Textilien und enthalten bevorzugt Wirkstoffe, die zu einem weichen Griff der behandelten Textilen führen, insbesondere kationischen Wirkstoffe (vorzugsweise Kationtenside, z.B. quartäre Ammonium-Verbindungen), Fettsäure-Derivate und/oder Siliconöle. Duftspüler sind parfümhaltige Textilnachbehandlungsmittel zur Pflege von Textilien, die einen besonders angenehmen Duft der Textilien bewirken. Konditioniertücher für die Anwendung im Wäschetrockner sind mit Wirkstoffen (insbesondere Weichspülern) imprägnierte Vliese oder Tücher (sheets). Hygienespüler sind Textilnachbehandlungsmittel zur Pflege von Textilien, welche wenigstens einen antimikrobiellen Wirkstoff, z.B. quartäre Ammonium-Verbindungen wie z.B. Benzalkoniumchlorid, enthalten und der Verringerung der Keimbelastung der Wäsche dienen.

Der Begriff der Reinigungsmittel umfasst alle Reiniger für harte oder weiche Oberflächen, vorzugsweise aber harte Oberflächen, wobei insbesondere Geschirrspülmittel (umfassend Handgeschirrspülmittel und Maschinengeschirrspülmittel), Allzweckreiniger, WC-Reiniger, Sanitärreiniger sowie Glasreiniger zu nennen sind.

Alle Wasch- oder Reinigungsmittel können z.B. in Form von Pulvern, Granulaten, Perlen, Tabletten, Pasten, Gelen, Tüchern, Stücken oder Flüssigkeiten vorliegen. Sie können einphasig oder mehrphasig sein. Sie können auch in Portionspackungen vorliegen, sogenannten Pouches.

Es ist besonders bevorzugt, dass die Wasch- oder Reinigungsmittel zumindest Tenside und/oder Gerüststoffe enthalten.

Als Tenside kommen insbesondere anionische Tenside, nichtionische Tenside, kationische, zwitterionische und/oder amphotere Tenside in Frage. Besonders bevorzugt ist es jedoch, wenn das erfindungsgemäße Wasch- oder Reinigungsmittel, anionische, nichtionische und/oder kationische Tenside enthält. Insbesondere der Einsatz einer Mischung aus anionischen und nichtionischen Tensiden ist vorteilhaft. Das erfindungsgemäße Wasch- oder Reinigungsmittel enthält 1 bis 40 Gew.-%, in weiter vorteilhafter Weise 3 bis 30 Gew.-% und insbesondere 5 bis 20 Gew.-% Tensid(e), insbesondere aus den Gruppen der anionischen Tenside, nichtionischen Tenside, kationischen, zwitterionischen und/oder amphotere Tenside. Dies entspricht einer bevorzugten Ausführungsform der Erfindung und ermöglicht optimale Reinigungsleistungen.

Bevorzugt einsetzbare Tenside werden jetzt folgend sowie weiter unten noch genauer beschrieben.

Besonders bevorzugt ist es, wenn das erfindungsgemäße Wasch- oder Reinigungsmittel Aniontensid enthält, vorteilhafterweise in Mengen von 0,1 bis 25 Gew. %, in weiter vorteilhafter Weise 1 bis 20 Gew-%, insbesondere in Mengen von 3 bis 15 Gew.-%, bezogen auf das gesamte Mittel. Dies entspricht einer bevorzugten Ausführungsform der Erfindung und ermöglicht besonders vorteilhafte Reinigungsleistungen. Ein besonders geeignetes Aniontensid ist Alkylbenzolsulfonat, vorzugsweise lineares Alkylbenzolsulfonat (LAS). Wenn das erfindungsgemäße Wasch- oder Reinigungsmittel Alkylbenzolsulfonat enthält, vorteilhafterweise in Mengen von 0,1 bis 25 Gew. %, in weiter vorteilhafter Weise 1 bis 20 Gew-%, insbesondere in Mengen von 3 bis 15 Gew.-%, bezogen auf das gesamte Mittel, so liegt eine bevorzugte Ausführungsform der Erfindung vor.

Besonders geeignete Aniontenside sind ferner die Alkylsulfate, insbesondere die Fettalkoholsulfate (FAS), wie z.B. C₁₂- bis C₁₈-Fettalkoholsulfat. Vorzugsweise können C₈- bis C₁₈-Alkylsulfate eingesetzt werden, besonders bevorzugt sind C₁₃-Alkylsulfat sowie C₁₃₋₁₅-Alkylsulfat und C₁₃₋₁₇-Alkylsulfat, vorteilhafterweise verzweigtes, insbesondere Alkyl-verzweigtes C₁₃₋₁₇-Alkylsulfat. Besonders geeignete Fettalkoholsulfate leiten sich vom Lauryl- und Myristylalkohol ab, sind also Fettalkoholsulfate mit 12 bzw. 14 Kohlenstoffatomen. Wenn das erfindungsgemäße Wasch- oder Reinigungsmittel Alkylsulfat, insbesondere C₁₂- bis C₁₈-Fettalkoholsulfat, enthält, vorteilhafterweise in Mengen von 0,1 bis 25 Gew. %, in weiter vorteilhafter Weise 1 bis 20 Gew-%, insbesondere in Mengen von 3 bis 15 Gew.-%, bezogen auf das gesamte Mittel, so liegt eine bevorzugte Ausführungsform der Erfindung vor.

Andere bevorzugt einsetzbare Aniontenside sind z.B. Alkansulfonate (z.B. sekundäres C13-C18-Alkansulfonat), Methylestersulfonate (z.B. α-C12-C18-Methylestersulfonat) und α-Olefinsulfonate (z.B. α-C14-C18-Olefinsulfonat) und Alkylethersulfate (z.B. C12-C14-Fettalkohol-2EO-ethersulfat) und/oder Seifen. Weitere geeignete Aniontenside werden weiter unten noch beschrieben. Besonders geeignet sind aber FAS und/oder LAS.

Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Besonders bevorzugt ist es, wenn das erfindungsgemäße Wasch- oder Reinigungsmittel Niotensid enthält, vorteilhafterweise in Mengen von 0,01 bis 25 Gew.-%, in weiter vorteilhafter Weise 1 bis 20 Gew.-%, insbesondere in Mengen von 3 bis 15 Gew.-%, bezogen auf das gesamte Mittel. Dies entspricht einer bevorzugten Ausführungsform der Erfindung. Besonders bevorzugt ist der Einsatz von Alkylpolyglycolethern, insbesondere in Kombination mit Aniontensid, wie vorzugsweise LAS.

Weitere geeignete Niotenside sind Alkylphenolpolyglykolether (APEO), (ethoxylierte) Sorbitanfettsäureester (Sorbitane), Alkylpolyglucoside (APG), Fettsäureglucamide, Fettsäureethoxylate, Aminoxide, Ethylenoxid-Propylenoxid-Blockpolymere, Polyglycerolfettsäureester und/oder Fettsäurealkanolamide. Weitere geeignete Niotenside werden weiter unten noch beschrieben. Niotenside auf Zuckerbasis, wie insbesondere APG, sind besonders bevorzugt.

Zu den vorzugsweise einsetzbaren Gerüststoffen im Sinne der Erfindung zählen insbesondere Polycarboxylate, Citrate (wie z.B. Natriumcitrat), Soda, Natriumhydrogencarbonat, Phosphate, Natriumsilicate (Wasserglas), Phosphonate, Zeolithe, alkalische amorphe Disilicate sowie kristalline Schichtsilicate. Gerüststoffe sind in dem erfindungsgemäßen Wasch- oder Reinigungsmittel vorzugsweise in Mengen von 0,1 bis 80 Gew.-%, vorteilhafterweise 1 bis 60 Gew.-%, in weiter vorteilhafter Weise, 5 bis 50 Gew.-% enthalten.

Weiterhin ist es ganz besonders bevorzugt, dass das erfindungsgemäße Wasch- oder Reinigungsmittel ein Buildersystem (d.h. zumindest 2 Substanzen mit Builderwirkung) enthält, vorzugsweise ein zeolithhaltiges Buildersystem, vorzugsweise umfassend Zeolith in Mengen > 1 Gew.-%, noch vorteilhafter > 5 Gew.-%, weiter vorteilhaft > 10 Gew.- %, insbesondere ≥ 15 Gew.-%, Gew.-% bezogen auf das gesamte Mittel. Eine sinnvolle Obergrenze an Zeolith kann z.B. bei 40 Gew.-%, 30 Gew.-% oder 20 Gew.-% liegen, bezogen auf das gesamte Mittel. Dies entspricht einer bevorzugten Ausführungsform der Erfindung. Bevorzugt ist Kombination von Zeolith mit Soda. Die Begriffe Builder und Gerüststoff sind synonym.

Ebenfalls ist es besonders bevorzugt, wenn das erfindungsgemäße Wasch- oder Reinigungsmittel ein lösliches Buildersystem, vorzugsweise umfassend Soda, Silikat, Citrat und/oder Polycarboxylate, enthält, vorteilhafterweise in Mengen von 0,1 bis 50 Gew.-%, bezogen auf das gesamte Mittel. Dies entspricht einer bevorzugten Ausführungsform der Erfindung. Ist ein solches lösliches Buildersystem enthalten, so ist es überaus bevorzugt, wenn nur geringe Mengen unlöslicher Builder, wie insbesondere Zeolith, z.B. < 5 Gew.-% bis 0,1 Gew.-% enthalten sind, insbesondere in solchem Falle gar kein unlöslicher Builder enthalten ist.

Ebenfalls ist es möglich, dass das erfindungsgemäße Wasch- oder Reinigungsmittel Phosphate enthält, wobei Phosphat vorzugsweise in Mengen von 1 bis 40 Gew.-%, insbesondere 5 bis 30 Gew.-% enthalten ist, bezogen auf das gesamte Mittel. Nach einer anderen bevorzugten Ausführungsform ist das erfindungsgemäße Wasch- oder Reinigungsmittel jedoch frei von Phosphaten.

Die erfindungsgemäßen Wasch- oder Reinigungsmittel, die z.B. als insbesondere pulverförmige Feststoffe, in nachverdichteter Teilchenform, als homogene Lösungen oder Suspensionen vorliegen können, können weiterhin im Prinzip alle bekannten und in derartigen Mitteln üblichen Inhaltsstoffe enthalten. Die erfindungsgemäßen Mittel können, wie schon gezeigt wurde, insbesondere Buildersubstanzen, Tenside, weiterhin auch Bleichmittel, Bleichaktivatoren, wassermischbare organische Lösungsmittel, Enzyme, Sequestrierungsmittel, Elektrolyte, pH-Regulatoren und weitere Hilfsstoffe, wie optische Aufheller, Fluoreszenzmittel, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Glaskorrosionsinhibitoren, Desintegrationshilfsmittel Antistatika, Bittermittel, Bügelhilfsmittel, Phobier- und Imprägniermittel, Quell- und Schiebefestmittel, neutrale Füllsalze sowie UV-Absorber, Schaumregulatoren sowie Farb- und Riechstoffe enthalten.

Im Folgenden werden einsetzbare Inhaltsstoffe zum Teil noch weiter erläutert.

Organische Buildersubstanzen können gemäß einer bevorzugten Ausführungsform der Erfindung eingesetzt werden und können gewünschtenfalls in Mengen z.B. bis zu 40 Gew.-%, insbesondere bis zu 25 Gew.-% und vorzugsweise von 1 bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt.

Als wasserunlösliche, wasserdispergierbare anorganische Buildermaterialien können insbesondere kristalline oder amorphe Alkalialumosilikate, in Mengen von bis zu 50 Gew.-%, vorzugsweise nicht über 40 Gew.-% und in flüssigen Mitteln insbesondere von 1 bis 5 Gew.-%, eingesetzt werden. Unter diesen sind die kristallinen Natriumalumosilikate in Waschmittelqualität, insbesondere Zeolith A, P und gegebenenfalls X, allein oder in Mischungen, beispielsweise in Form eines Co-Kristallisats aus den Zeolithen A und X (Vegobond® AX, ein Handelsprodukt der Condea Augusta S.p.A.), bevorzugt. Mengen nahe der genannten Obergrenze werden vorzugsweise in festen, teilchenförmigen Mitteln eingesetzt.

Als für den Einsatz in erfindungsgemäßen Wasch- oder Reinigungsmittel geeignete Bleichmittel kommen z.B. Persauerstoffverbindungen wie insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Falls ein erfindungsgemäßes Mittel Persauerstoffverbindungen enthält, sind diese in Mengen von vorzugsweise bis zu 50 Gew.-%, insbesondere von 5 bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bleichaktivatoren können, insbesondere bei Anwesenheit obengenannter Wasserstoffperoxid-liefernder Bleichmittel, im üblichen Mengenbereich, vorzugsweise in Mengen von 0,5 bis 10 Gew.-%, insbesondere 1 bis 8 Gew.-%, bezogen auf gesamtes Mittel, enthalten sein, fehlen bei Einsatz von Percarbonsäure als alleinigem Bleichmittel jedoch vorzugsweise ganz.

Als in den erfindungsgemäßen Wasch- oder Reinigungsmittel verwendbare Enzyme kommen vor allem solche aus der Klasse der Amylasen, Proteasen, Lipasen, Cutinasen, Pullulanasen, Hemicellulasen, Cellulasen, Oxidasen, Laccasen, Pektinasen, Carboanhydrasen, Mannanasen, Tannasen und Peroxidasen sowie deren Gemische in Frage.

Zu den in den erfindungsgemäßen Wasch- oder Reinigungsmittel, insbesondere wenn sie in flüssiger oder pastöser Form vorliegen, neben Wasser verwendbaren organischen Lösungsmitteln gehören Alkohole mit 1 bis 4 C-Atomen, insbesondere Methanol, Ethanol, Isopropanol und tert-Butanol, Diole mit 2 bis 4 C-Atomen, insbesondere Ethylenglykol und Propylenglykol, sowie deren Gemische und die aus den genannten Verbindungsklassen ableitbaren Ether.

Zur Einstellung eines gewünschten, sich durch die Mischung der übrigen Komponenten nicht von selbst ergebenden pH-Werts können die erfindungsgemäßen Wasch- oder Reinigungsmittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren können in den erfindungsgemäßen Mitteln in Mengen von vorzugsweise nicht über 20 Gew.-%, insbesondere von 1,2 bis 17 Gew.-%, optional enthalten sein.

Insbesondere beim Einsatz in maschinellen Verfahren kann es von Vorteil sein, den Wasch- oder Reinigungsmittel übliche Schauminhibitoren zuzusetzen. Als Schauminhibitoren eignen sich beispielsweise Seifen natürlicher oder synthetischer Herkunft, die einen hohen Anteil an C₁₈- bis C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden.

Um den ästhetischen Eindruck der Wasch- oder Reinigungsmittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Wasch- oder Reinigungsmittel und gegen Licht sowie keine ausgeprägte Substantivität gegenüber Textilfasern, um diese nicht anzufärben.

Geeignete Soil-Release-Polymere, die auch als "Antiredepositionsmittel" bezeichnet werden, sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether sowie die aus dem Stand der Technik bekannten Polymere der Phthalsäure und/oder Terephthalsäure bzw. von deren Derivaten, insbesondere Polymere aus Ethylenterephthalaten und/oder Polyethylen- und/oder Polypropylenglykolterephthalaten oder anionisch und/oder nichtionisch modifizierten Derivaten von diesen. Geeignete Derivate umfassen die sulfonierten Derivate der Phthalsäure- und Terephthalsäure-Polymere.

Optische Aufheller (so genannte "Weißtöner") können den Wasch- oder Reinigungsmittel zugesetzt werden, um Vergrauungen und Vergilbungen der behandelten Textilen Flächengebilden zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate. Die optischen Aufheller werden üblicherweise in Mengen zwischen 0% und 0,3 Gew.-%, bezogen auf das fertige Mittel, optional eingesetzt.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die oben genannten Stärkeprodukte verwenden, zum Beispiel abgebaute Stärke, Aldehydstärken usw. Auch Polyvinylpyrrolidon ist brauchbar. Bevorzugt werden jedoch Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxymethylcellulose und deren Gemische in Mengen von 0,1 bis 5 Gew.-%, bezogen auf die Wasch- oder Reinigungsmittel, optional eingesetzt.

Um während des Waschens und/oder des Reinigens von gefärbten Textilien die Farbstoffablösung und/oder die Farbstoffübertragung auf andere Textilien wirksam zu unterdrücken, kann das Wasch- oder Reinigungsmittel einen Farbübertragungsinhibitor enthalten. Es ist bevorzugt, dass der Farbübertragungsinhibitor ein Polymer oder Copolymer von cyclischen Aminen wie beispielsweise Vinylpyrrolidon und/oder Vinylimidazol ist.

Die Menge an optionalem Farbübertragungsinhibitor bezogen auf die Gesamtmenge des Wasch- oder Reinigungsmittels liegt bevorzugt von 0,01 bis 2 Gew.-%, vorzugsweise von 0,05 bis 1 Gew.-% und mehr bevorzugt von 0,1 bis 0,5 Gew.-%.

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können die Wasch- oder Reinigungsmittel synthetische Knitterschutzmittel enthalten. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Zur Bekämpfung von Mikroorganismen können die Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat, wobei bei den erfindungemäßen Wasch- oder Reinigungsmitteln auch gänzlich auf diese Verbindungen verzichtet werden kann.

Die erfindungsgemäßen Wasch- oder Reinigungsmittel können Konservierungsmittel enthalten, wobei vorzugsweise nur solche eingesetzt werden, die kein oder nur ein geringes hautsensibilisierendes Potential besitzen. Beispiele sind Sorbinsäure und seine Salze, Benzoesäure und seine Salze, Salicylsäure und seine Salze, Phenoxyethanol, 3-lodo-2-propynylbutylcarbamat, Natrium N-(hydroxymethyl)glycinat, Biphenyl-2-ol sowie Mischungen davon. Ein geeignetes Konservierungsmittel stellt die lösungsmittelfreie, wässrige Kombination von Diazolidinylharnstoff, Natriumbenzoat und Kaliumsorbat (erhältlich als Euxyl® K 500 ex Schuelke & Mayr) dar, welches in einem pH-Bereich bis 7 eingesetzt werden kann. Insbesondere eignen sich Konservierungsmittel auf Basis von organischen Säuren und/oder deren Salzen zur Konservierung der erfindungsgemäßen, hautfreundlichen Wasch- oder Reinigungsmittel.

Um unerwünschte, durch Sauerstoffeinwirkung und andere oxidative Prozesse verursachte Veränderungen an den Wasch- oder Reinigungsmitteln und/oder den behandelten textilen Flächengebilden zu verhindern, können die Wasch- oder Reinigungsmittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite, Phosphonate und Vitamin E.

Ein erhöhter Tragekomfort kann aus der zusätzlichen Verwendung von Antistatika resultieren, die den Wasch- oder Reinigungsmitteln zusätzlich beigefügt werden können.

Schließlich können die Wasch- oder Reinigungsmittel auch UV-Absorber enthalten, die auf die behandelten textilen Flächengebilde aufziehen und die Lichtbeständigkeit der Fasern verbessern.

Um die durch Schwermetalle katalysierte Zersetzung bestimmter Waschmittel-Inhaltsstoffe zu vermeiden, können Stoffe eingesetzt werden, die Schwermetalle komplexieren. Geeignete Schwermetallkomplexbildner sind beispielsweise die Alkalisalze der Ethylendiamintetraessigsäure (EDTA) oder Alkalimetallsalze von anionischen Polyelektrolyten wie Polymaleaten und Polysulfonaten.

Eine bevorzugte Klasse von Komplexbildnern sind die Phosphonate, die in bevorzugten Wasch- oder Reinigungsmittel in Mengen von z.B. 0,01 bis 2,5 Gew.-%, vorzugsweise 0,02 bis 2 Gew.-% und insbesondere von 0,03 bis 1,5 Gew.-% optional enthalten sind. Zu diesen bevorzugten Verbindungen zählen insbesondere Organophosphonate wie beispielsweise 1-Hydroxyethan-1,1-diphosphonsäure (HEDP), Aminotri(methylenphosphonsäure) (ATMP), Diethylentriamin-penta-(methylenphosphonsäure) (DTPMP bzw. DETPMP) sowie 2-Phosphonobutan-1,2,4-tricarbonsäure (PBS-AM), die zumeist in Form ihrer Ammonium- oder Alkalimetallsalze eingesetzt werden.

Feste Wasch- oder Reinigungsmittel können zusätzlich z.B. noch neuträle Füllsalze wie Natriumsulfat enthalten.

Flüssige Wasch- oder Reinigungsmittel können zusätzlich z.B. noch Verdickungsmittel enthalten, z.B. um eine gewünschte Viskosität einzustellen. Geeignete und einsetzbare Verdickungsmittel werden auch weiter unten im Zusammenhang mit den Textilnachbehandlungsmitteln beschrieben. Die dort genannten Verdickungsmittel können auch in allen anderen flüssigen Wasch- oder Reinigungsmitteln eingesetzt werden.

Die erfindungsgemäßen Wasch- und Reinigungsmittel können insbesondere Parfümöle (Riechstoffe) enthalten. Dies entspricht einer besonders bevorzugten Ausführungsform der Erfindung. Bezogen auf das gesamte Wasch- oder Reinigungsmittel können darin vorzugsweise 0,0001 bis 15 Gew.-%, vorteilhafterweise 0,001 bis 10 Gew.-%, insbesondere 0,01 bis 5 Gew.-% Riechstoffe enthalten sein.

Als Duftstoffe bzw. Riechstoffe bzw. Parfümöle können alle dafür bekannten Stoffe und Gemische eingesetzt werden. Im Sinne dieser Erfindung werden die Begriffe "Riechstoff(e)", "Duftstoffe" und "Parfümöl(e)" synonym gebraucht. Damit sind insbesondere all jene Stoffe oder deren Gemische gemeint, die von Mensch und Tier als Geruch empfunden werden, insbesondere vom Mensch als Wohlgeruch empfunden werden.

Als Duftkomponenten können Parfüme, Parfümöle oder Parfümölbestandteile eingesetzt werden. Parfümöle bzw. Duftstoffe können erfindungsgemäß einzelne Riechstoffverbindungen, z.B. die synthetischen Produkte vom Typ der Ester, Ether, Aldehyde, Ketone, Alkohole und Kohlenwasserstoffe sein.

Ein bevorzugtes erfindungsgemäßes, pulverförmiges Vollwaschmittel kann neben den erfindungsgemäßen Hydrogelbildnern vorzugsweise z.B. Komponenten enthalten, die ausgewählt sind aus den folgenden:
- Aniontenside, wie z.B. Alkylbenzolsulfonat und/oder Alkylsulfat, in Mengen von z.B. 0 bis 40 Gew.-%, vorteilhafterweise 5 bis 30 Gew.-%, vorzugsweise 8 bis 25 Gew.-%, insbesondere 10 bis 20 Gew.-%,
- Nichtionische Tenside, wie z.B. Fettalkoholpolyglycolether, Alkylpolyglucosid und/oder Fettsäureglucamid, in Mengen von z.B. 0 bis 30 Gew.-%, vorteilhafterweise 0,1 bis 20 Gew.-%, vorzugsweise 2 bis 15 Gew.-%, insbesondere 6 bis 11 Gew.-%,
- Gerüststoffe, wie z.B. Zeolith, Polycarboxylat und/oder Natriumcitrat, in Mengen von z.B. 0 bis 70 Gew.-%, vorteilhafterweise 5 bis 60 Gew.-%, vorzugsweise 10 bis 55 Gew.-%, insbesondere 15 bis 40 Gew.-%,
- Alkalien, in Mengen von z.B. 0 bis 35 Gew.-% vorteilhafterweise 1 bis 30 Gew.-%, vorzugsweise 2 bis 25 Gew.-%, insbesondere 5 bis 20 Gew.-%,
- Bleichmittel, wie z.B. Natriumperborat und/oder Natriumpercarbonat, in Mengen von z.B. 0 bis 30 Gew.-% vorteilhafterweise 5 bis 25 Gew.-%, vorzugsweise 10 bis 20 Gew.-%,
- Korrosionsinhibitoren, z.B. Natriumsilicat, in Mengen von z.B. 0 bis 10 Gew.-%, vorteilhafterweise 1 bis 6 Gew.-%, vorzugsweise 2 bis 5 Gew.-%, insbesondere 3 bis 4 Gew.-%,
- Stabilisatoren, z.B. Phosphonate, vorteilhafterweise 0 bis 1 Gew.-%,
- Schauminhibitor, z.B. Seife, Siliconöle und/oder Paraffine vorteilhafterweise 0 bis 4 Gew.-%, vorzugsweise 0,2 bis 3 Gew.-%, insbesondere 0,5 bis 1,5 Gew.-%,
- Enzyme, z.B. Proteasen, Amylasen, Cellulasen und/oder Lipasen, vorteilhafterweise 0 bis 2 Gew.-%, vorzugsweise 0,2 bis 1 Gew.-%, insbesondere 0,3 bis 0,8 Gew.-%,
- Vergrauungsinhibitor, z.B. Carboxymethylcellulose, vorteilhafterweise 0 bis 1 Gew.-%,
- Verfärbungsinhibitor, z.B. Polyvinylpyrrolidon-Derivate, insbesondere 0 bis 2 Gew.-%,
- Stellmittel, z.B. Natriumsulfat, vorteilhafterweise 0 bis 20 Gew.-%,
- Optische Aufheller, z.B. Stilben-Derivat und/oder Biphenyl-Derivat, vorteilhafterweise 0,1 bis 0,4 Gew.-%, insbesondere 0,1 bis 0,3 Gew.-%,
- Riechstoffe,
- Wasser,
- Seife,
- Bleichaktivatoren,
- Cellulosederivate,
- Schmutzabweiser,
Gew.-% jeweils bezogen auf das gesamte Mittel.

Ein bevorzugtes, erfindungsgemäßes flüssiges Vollwaschmittel kann neben den erfindungsgemäßen Hydrogelbildnern vorzugsweise Komponenten enthalten, die ausgewählt sind aus den folgenden:
- Aniontenside, wie z.B. Alkylbenzolsulfonat und/oder Alkylsulfat, in Mengen von z.B. 0 bis 40 Gew.-%, vorteilhafterweise 5 bis 40 Gew.-%, vorzugsweise 8 bis 30 Gew.-%, insbesondere 10 bis 25 Gew.-%,
- Nichtionische Tenside, wie z.B. Fettalkoholpolyglycolether, Alkylpolyglucosid und/oder Fettsäureglucamid, in Mengen von z.B. 0 bis 30 Gew.-%, vorteilhafterweise 0,1 bis 25 Gew.-%, vorzugsweise 5 bis 20 Gew.-%, insbesondere 10 bis 15 Gew.-%,
- Gerüststoffe, wie z.B. Zeolith, Polycarboxylat und/oder Natriumcitrat, vorteilhafterweise 0 bis 15 Gew.-%, vorzugsweise 0,01 bis 10 Gew.-%, insbesondere 0,1 bis 5 Gew.-%,
- Schauminhibitor, z.B. Seife, Siliconöle und/oder Paraffine, in Mengen von z.B. 0 bis 10 Gew.-%, vorteilhafterweise 0,1 bis 4 Gew.-%, vorzugsweise 0,2 bis 2 Gew.-%, insbesondere 1 bis 3 Gew.-%,
- Enzyme, z.B. Proteasen, Amylasen, Cellulasen und/oder Lipasen, in Mengen von z.B. 0 bis 3 Gew.-%, vorteilhafterweise 0,1 bis 2 Gew.-%, vorzugsweise 0,2 bis 1 Gew.-%, insbesondere 0,3 bis 0,8 Gew.-%,
- Optische Aufheller, z.B. Stilben-Derivat und/oder Biphenyl-Derivat, in Mengen von z.B. 0 bis 1 Gew.-%, vorteilhafterweise 0,1 bis 0,4 Gew.-%, insbesondere 0,1 bis 0,3 Gew.-%,
- Riechstoffe,
- Stabilisatoren,
- Wasser,
- Seife, in Mengen von z.B. 0 bis 25 Gew.-%, vorteilhafterweise 1 bis 20 Gew.-%, vorzugsweise 2 bis 15 Gew.-%, insbesondere 5 bis 10 Gew.-%,
- Alkohole/Lösungsmittel, vorteilhafterweise 0 bis 25 Gew.-%, vorzugsweise 1 bis 20 Gew.-%, insbesondere 2 bis 15 Gew.-%, Gew.-% jeweils bezogen auf das gesamte Mittel.

Ein weiterer Gegenstand der Erfindung ist ein Textilwaschverfahren, unter Einsatz eines erfindungsgemäßen Wasch- oder Reinigungsmittels (wie zuvor beschrieben), vorzugsweise in einer automatischen Waschmaschine, wobei die Waschtemperatur ≤ 60°C, vorzugsweise ≤ 40°C beträgt.

Bevorzugte erfindungsgemäße Wasch- oder Reinigungsmittel sind Textilnachbehandlungsmittel. Dabei handelt es sich vorzugsweise um Weichspüler, also um Textilnachbehandlungsmittel, die eine weichmachende Komponente enthalten. Bevorzugt enthaltene Wirkstoffe in den erfindungsgemäßen Weichspülerformulierungen sind Kationtenside, insbesondere Esterquats. Esterquats sind quartäre Ammonium-Verbindungen mit vorzugsweise zwei hydrophoben Resten, die jeweils eine Ester-Gruppe als sogenannte Sollbruchstelle für einen leichteren biologischen Abbau enthalten.

Wenn das erfindungsgemäße Textilnachbehandlungsmittel also eine weichmachende Verbindung enthält, wobei die Menge an weichmachender Verbindung vorzugsweise 2 bis 80 Gew.-%, vorteilhafterweise 4 bis 40 Gew.-%, weiter bevorzugt 6 bis 20 Gew.-% und insbesondere 8 bis 15 Gew.-%, jeweils bezogen auf das gesamte Mittel, beträgt, so liegt eine bevorzugte Ausführungsform der Erfindung vor. Wenn ein Kationtensid enthalten ist, vorteilhafterweise eine quartäre AmmoniumVerbindung, insbesondere Esterquat, vorzugsweise in Mengen von > 0,1 Gew.-%, vorteilhafterweise 1 bis 40 Gew.-%, insbesondere 3 bis 30 Gew.-%, bezogen auf das gesamte Mittel, so liegt eine weitere bevorzugte Ausführungsform der Erfindung vor.

Nachbehandlungsmittel werden üblicherweise im letzten Schritt eines konventionellen Textilwaschvorgangs, dem Spülgang, in Kontakt mit den Textilien gebracht. Die Nachbehandlung kann auch im Wäschetrockner erfolgen und zwar insbesondere durch den Einsatz der zuvor genannten Trocknertücher.

Die weichmachende Komponente umfasst beispielsweise quaternäre Ammoniumverbindungen wie Monoalk(en)yltrimethylammonium-Verbindungen, Dialk(en)yldimethylammonium-Verbindungen, Mono-, Di- oder Triester von Fettsäuren mit Alkanolaminen.

Besonders bevorzugte weichmachende Komponenten sind alkylierte quaternäre Ammoniumverbindungen, von denen mindestens eine Alkylkette durch eine Estergruppe und/oder Amidogruppe unterbrochen ist. Ganz besonders bevorzugt sind N-Methyl-N-(2-hydroxyethyl)-N,N-(ditalgacyloxy-ethyl)ammonium-methosulfat oder Bis-(palmitoyloxyethyl)-hydroxyethyl-methyl-ammonium-methosulfat.
Weitere erfindungsgemäß verwendbare weichmachende Komponenten stellen quaternisierten Proteinhydrolysate oder protonierte Amine dar.

Weiterhin sind auch kationische Polymere geeignete weichmachende Komponente. Zu den geeigneten kationischen Polymeren zählen die Polyquaternium-Polymere, wie sie im CTFA Cosmetic Ingredient Dictionary (The Cosmetic, Toiletry and Fragrance, Inc., 1997), insbesondere die auch als Merquats bezeichneten Polyquaternium-6-, Polyquaternium-7-, Polyquaternium-10-Polymere (Polymer JR, LR und KG Reihe von Amerchol), Polyquaternium-4-Copolymere, wie Pfropfcopolymere mit einen Cellulosegerüst und quartären Ammoniumgruppen, die über Allyldimethylammoniumchlorid gebunden sind, kationische Cellulosederivate, wie kationisches Guar, wie Guarhydroxypropyltriammoniumchlorid, und ähnliche quaternierte Guar-Derivate (z.B. Cosmedia Guar von Cognis oder die Jaguar Reihe von Rhodia), kationische quaternäre Zuckerderivate (kationische Alkylpolyglucoside), z.B. das Handelsprodukt Glucquat® 100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosiliconpolymere und Copolymere.

Ebenfalls einsetzbar sind polyquaternierte Polymere (z.B. Luviquat® Care von BASF) und auch kationische Biopolymere auf Chitinbasis und deren Derivate, beispielsweise das unter der Handelsbezeichnung Chitosan® (Hersteller: Cognis) erhältliche Polymer.

Weitere geeignete weichmachende Komponenten umfassen protonierte oder quaternierte Polyamine.

Das Textilnachbehandlungsmittel, wie auch die Gesamtheit der Wasch- oder Reinigungsmittel, kann ferner wenigstens eine Aromatherapiekomponente enthalten. Als Aromatherapiekomponente ist bevorzugt ein ätherisches Öl einsetzbar. Ätherisches Öl fällt auch unter den Oberbegriff der Riechstoffe.

Die Menge an ätherischem Öl in dem Wasch- oder Reinigungsmittel, vorzugsweise Textilnachbehandlungsmittel, beträgt vorzugsweise von 0,0001 bis 3 Gew.-%, insbesondere bevorzugt von 0,01 bis 1 Gew.-% und ganz besonders bevorzugt von 0,05 bis 0,5 Gew.-%.

Ein bevorzugtes erfindungsgemäßes Textilnachbehandlungsmittel umfasst neben den erfindungsgemäßen Hydrogelbildnern insbesondere
a) weichmachende Verbindung, vorteilhafterweise Kationtenside, vorzugsweise Esterquats, vorteilhafterweise in Mengen von 5 bis 30 Gew.-%, z.B. 10 bis 20 Gew.-%,
b) Tenside, vorzugsweise Niotenside, z.B. Fettalkoholethoxylate, vorteilhafterweise in Mengen von 0 bis 5 Gew.-%, z.B. 0,1 bis 3 Gew.-%.
c) Konservierungsmittel, vorteilhafterweise in Mengen von 0 bis 2 Gew.-%, z.B. 0,001 bis 0,5 Gew.-%,
d) Riechstoffe, vorteilhafterweise in Mengen von 0 bis 10 Gew.-% oder 0 bis 5 Gew.-%, z.B. 0,01 bis 1 Gew.-%,
e) Farbstoffe, vorteilhafterweise in Mengen von 0 bis 0,1 Gew.-%, z.B. 0,01 bis 0,005 Gew.-%,
f) optional Wasser, vorzugsweise in Mengen ≥ 50 Gew.-% oder ≥ 60 Gew.-%, z.B. 70 bis 95 Gew.-% oder z.B. 75 bis 90 Gew.-%.
g) optional Lösungsmittel, vorzugsweise einwertige Alkohole, insbesondere 2-Propanol, vorteilhafterweise in Mengen von 0,05 bis 5 Gew.-%, insbesondere 0,1 bis 4 Gew.-%, insbesondere 0,3 bis 3 Gew.-%,
h) optional pH-Stellmittel, vorzugsweise 0,01 bis 5 Gew.-%, insbesondere 0,02 bis 1 Gew.-%
i) optional Elektrolyte, vorzugsweise aus der Gruppe der anorganischen Salze, vorteilhafterweise MgCl₂ oder NaCl, 0,01 bis 5 Gew.-%, insbesondere 0,05 bis 2 Gew.-%,
j) optional hautpflegende Aktivstoffe, wie z.B. Mandelöl, vorzugsweise in einer Menge von 0 bis 15 Gew.-%, z.B. 0,1 bis 10 Gew.-%, insbesondere 0,5 bis 5 Gew.-%,
k) optional Verdicker, z.B. auf Polyacrylat-Basis, vorzugsweise in Mengen von 0,01 bis 3 Gew.-%, insbesondere 0,1 bis 1 Gew.-%,
Gew.-% jeweils bezogen auf das gesamte Mittel.

Bevorzugte Mittel im Sinne der Erfindung sind auch die Reinigungsmittel, insbesondere Reiniger für harte Oberflächen.

Wenn das erfindungsgemäße Reinigungsmittel ausgewählt ist aus der Gruppe der Handgeschirrspülmittel, der Maschinengeschirrspülmittel, der Toilettenreiniger bzw. WC-Reiniger, der Rohrreinigungsmittel bzw. Abflussreiniger, der Universal- bzw. Allzweckreiniger, der Sanitärreiniger, der Backofenreiniger bzw. Grillreiniger, der Metallputzmittel, der Glasreiniger bzw. Fensterreiniger, der Reinigungshilfsmittel, der Fußbodenreinigungsmittel und der Spezialreinigungsmittel, so liegt eine bevorzugte Ausführungsform der Erfindung vor.

Auch im Zusammenhang mit den Reinigungsmitteln liegt ein Vorteil der Erfindung darin, eine retardierte und/oder gezielte Freisetzung von Aktivstoffen, wie z.B. Duftstoffen, aus den gebildeten Gelschichten zu ermöglichen. Dadurch wird ein oft angestrebter "slow-release"-Effekt bzw. "long-lasting"-Effekt und/oder eine zielgenaue Aktivstofffreisetzung direkt auf dem behandelten Objekt ermöglicht. Die gereinigte Oberfläche, z.B. ein Fußboden, duftet gleichmäßig über einen längeren Zeitraum oder Duftstoffe werden freigesetzt, wenn die eingelagerten Duftstoffe abgegeben werden. Ebenso können auch andere Aktivstoffe, wie z.B. Flüssigkeiten mit antimikrobiellen Wirkstoffen, Germiziden, Fungiziden oder anderen Aktivstoffen retardiert und/oder gezielt freigesetzt werden.

In einer bevorzugten Ausführungsform der Erfindung enthält ein erfindungsgemäßes Wasch- oder Reinigungsmittel, insbesondere Reinigungsmittel, ein anionisches Polymer, vorteilhafterweise in Mengen von 0,2 bis 30 Gew.-%, vorzugsweise 0,5 bis 20 Gew.-% und insbesondere 1 bis 12 Gew.-%, Gew.-% bezogen auf das gesamte Mittel.

Wenn als anionisches Polymer ein Copolymer, umfassend
i) ungesättigte Carbonsäure(n)
ii) Sulfonsäuregruppen-haltige(s) Monomer(e)
iii) weitere(s) nichtionogene(s) Monome(e)
enthalten ist, so liegt wiederum eine bevorzugte Ausführungsform der Erfindung vor.

Wenn das erfindungsgemäße Wasch- oder Reinigungsmittel, insbesondere Reinigungsmittel, Phosphonat enthält, vorzugsweise ausgewählt aus
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze,
so liegt eine weitere bevorzugte Ausführungsform der Erfindung vor.

Insbesondere Reinigungsmittel können Glaskorrosionsinhibitoren enthalten. Diese verhindern das Auftreten von Trübungen, Schlieren und Kratzern aber auch das Irisieren der Glasoberfläche von maschinell gereinigten Gläsern. Bevorzugte Glaskorrosionsinhibitoren stammen aus der Gruppe der Magnesium- und Zinksalze sowie der Magnesium- und Zinkkomplexe.

Mit besonderem Vorzug wird als Glaskorrosionsinhibitor mindestens ein Zinksalz einer organischen Carbonsäure, besonders bevorzugt ein Zinksalz aus der Gruppe Zinkstearat, Zinkoleat, Zinkgluconat, Zinkacetat, Zinklactat und Zinkcitrat eingesetzt. Auch Zinkricinoleat, Zinkabietat und Zinkoxalat sind bevorzugt.

Im Rahmen der vorliegenden Erfindung beträgt der Gehalt an optionalem Zinksalz in Reinigungsmitteln vorzugsweise zwischen 0,1 bis 5 Gew.-%, bevorzugt zwischen 0,2 bis 4 Gew.-% und insbesondere zwischen 0,4 bis 3 Gew.-%, bzw. der Gehalt an Zink in oxidierter Form (berechnet als Zn²⁺) zwischen 0,01 bis 1 Gew.-%, vorzugsweise zwischen 0,02 bis 0,5 Gew.-%, insbesondere zwischen 0,04 bis 0,2 Gew.-%, jeweils bezogen auf das Gesamtgewicht des glaskorrosionsinhibitorhaltigen Mittels.

Erfindungsgemäß bevorzugte Handgeschirrspülmittel können neben den erfindungsgemäßen Hydrogelbildnern z.B. enthalten:
a) Tenside, z.B. Alkansulfonate, Alkylethersulfate, Alkylpolyglucoside und/oder Cocoamidopropyl-Betain, vorzugsweise in Mengen von 5 bis 45 Gew.-%, insbesondere 10 bis 40 Gew.-%,
b) optional Säuerungsmittel, wie z.B. Citronensäure zur pH-Werteinstellung,
c) Hydrotrope, wie z.B. Cumolsulfonat, vorzugsweise in Mengen von 0 bis 15 Gew.-%, insbesondere 0,01 bis 10 Gew.-%,
d) Rückfetter, wie z.B. Fettsäureamide vorzugsweise in Mengen von 0 bis 3 Gew.-%, insbesondere 0,01 bis 3 Gew.-%,
e) Pflegekomponenten, wie z.B. Aloe vera-Extrakte, vorzugsweise in Mengen von 0 bis < 5Gew.-%, insbesondere 0,001 bis < 3 Gew.-%,
f) Riechstoffe, vorzugsweise in Mengen von 0 bis 3 Gew.-%, insbesondere 0,01 bis 2 Gew.-%,
g) optional Farbstoffe,
h) Antibakterielle Wirkstoffe, wie z.B. Natriumbenzoat oder Natriumsalicylat, vorzugsweise in Mengen von 0 bis 3 Gew.-%, insbesondere 0,001 bis 2 Gew.-%,
i) Konservierungsmittel, vorzugsweise in Mengen von 0 bis 1 Gew.-%, insbesondere 0,001 bis 0,5 Gew.-%.

Erfindungsgemäß bevorzugte Maschinengeschirrspülmittel können neben den erfindungsgemäßen Hydrogelbildnern z.B. Natriumphosphate, vorzugsweise Pentanatriumtriphosphat, Phosphonate, Citrate, vorzugsweise Natriumcitrat, Polycarboxylate, Natriummetasilicate, Soda, Natriumhydrogencarbonat, Natriumdisilicat, Aktivchlor, Natriumperborat, Bleichaktivator TAED, Enzyme, vorzugsweise Protease und Amylase, schaumarme nichtionische Tenside, Silber-/Glasschutz sowie Riechstoffe enthalten. Bevorzugte Maschinengeschirrspülmittel können z.B. phosphatbasiert und hochalkalisch oder aber z.B. phosphatbasiert und niederalkalisch sein. Andere bevorzugte Maschinengeschirrspülmittel können z.B. phosphatfrei und niederalkalisch sein.

Ein weiterer Gegenstand der Erfindung ist ein Verfahren zur Reinigung harter Oberfläche, unter Einsatz eines erfindungsgemäßen Reinigungsmittels, wie zuvor beschrieben, vorzugsweise in Verbindung mit Wasser.

Offenbart wird außerdem die Verwendung von erfindungsgemäßen Hydrogelbildnern in Wasch- oder Reinigungsmitteln zur Erzeugung von Hydrogelschichten auf harten oder textilen Oberflächen (durch die Oberflächen induziert).
Ein weiterer Gegenstand der Erfindung ist ein Verfahren zur Erzeugung von Hydrogelschichten auf Oberflächen, insbesondere harten oder weichen Oberflächen, wobei die betreffende Oberfläche mit einem erfindungsgemäßen Wasch- oder Reinigungsmittel behandelt wird oder mit einer Flüssigkeit, die einen erfindungsgemäßen Hydrogelbildner umfasst.

Es entspricht dabei einer bevorzugten Ausführungsform der Erfindung, wenn bei der Schichtbildung Aktivstoffe, wie insbesondere Riechstoffe, Pflegestoffe, antimikrobielle Wirkstoffe in die Schichten ein- bzw. an die Schichten angelagert werden.

Wenn das erfindungsgemäße Verfahren zur (a) Hydrophilierung von harten oder weichen Oberflächen, (b) Erleichterung der Entfernbarkeit von Schmutz von harten oder weichen Oberflächen, (c) Verbesserung des Schmutzabweisevermögens harter oder weicher Oberflächen (d) retardierten Freisetzung von Wirkstoffen, insbesondere Riechstoffen und antimikrobiellen Stoffen, welche in den Gelschichten gespeichert sind, und/oder (e) Verbesserung des Weichgriffes von Textilien vorgesehen ist, so liegt eine bevorzugte Ausführungsform der Erfindung vor.

Wenn das erfindungsgemäße Verfahren in einer automatischen Waschmaschine durchgeführt wird, vorzugsweise bei Temperaturen ≤ 40°C, insbesondere ≤ 30°C, so liegt eine bevorzugte Ausführungsform der Erfindung vor.

Ein weiterer Gegenstand der Erfindung ist ein Textilwaschverfahren, welches unter Einsatz eines erfindungsgemäßen Waschmittels durchgeführt wird. Das Verfahren wird vorzugsweise in einer automatischen Waschmaschine durchgeführt wird, vorzugsweise bei Temperaturen ≤ 40°C, insbesondere ≤ 30°C.

Ein weiterer Gegenstand der Erfindung ist ein Reinigungsverfahren für harte Oberflächen, welches unter Einsatz eines erfindungsgemäßen Reinigungsmittels durchgeführt wird.

### Beispiele:

### 1. Synthesebeispiel:

Es wurde der folgende erfindungsgemäße Hydrogelbildner (1,1,1,14,14,14-Tetradecandicarboxyethyltetracarboxamide, N1,N'1,N14,N'14,-tetrakis [2-hydroxy-1,1-bis(hydroxymethyl)ethyl]-) synthetisiert:

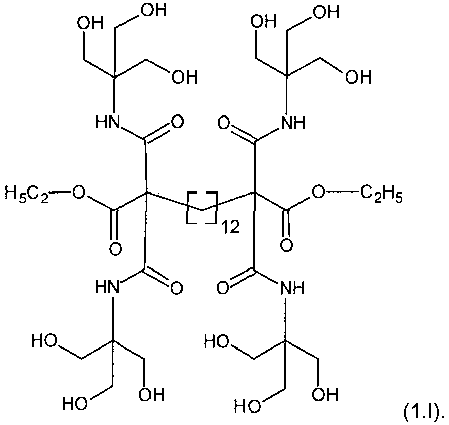

Dazu wurden 1,12-Dibromdodecan (1 Äq) und Triethylmethantricarboxylat (2 Äq) in Toluol und DMF unter Argon-Atmosphäre 24 h am Rückfluss gerührt. Die Reaktionsmischung wurde mit Wasser und NaHCO₃-Lösung gewaschen. Die organische Phase wurde getrocknet und eingeengt. Das erhaltene Produkt wurde in DMSO gelöst, und dazu wurden 7 Äq Na₂CO₃ gegeben. 6 Äq Tris(hydroxymethyl)-aminomethan wurden in DMSO gelöst und zum Gemisch dazugegeben. Der Ansatz wurde bei 80°C gerührt. Anschließend wurde das Lösungsmittel entfernt. Das Rohprodukt wurde mit Wasser und Aceton gereinigt.

Nach Esterhydrolyse konnte die folgende Verbindung erhalten werden:

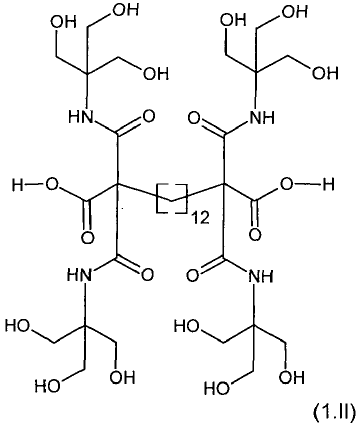

### 2. Anwendungsbeispiele:

### 2.1.

Unter Verwendung von Wasser wurde eine 0,05 Gew.-% wässrige Lösung des erfindungsgemäßen Hydrogelbildners aus dem eben genannten Synthesebeispiel (1.) hergestellt. In diese dünnflüssige Lösung wurde ein 25 mm x 50 mm großer Stoffstreifen (Polyester; Flächengewicht 130 g/m²) eingetaucht, wieder herausgenommen und getrocknet. Anschließend wurde das Textil mittels Rasterelektronenmikroskopie untersucht. Im Vergleich mit dem unbehandelten Gewebe konnte auf dem behandelten Gewebe deutlich die Ausbildung eines Gels, das aus Gelfibrillen aufgebaut war, nachgewiesen werden.

### 2.2.

Es wurde eine wässrige Lösung enthaltend 0,05 Gew.-% des erfindungsgemäßen Hydrogelbildners aus dem eben genannten Synthesebeispiel (1.), 0,044 Gew.-% lineares Alkylbenzolsulfonat, 0,044 Gew.-% Dehydol LT 7 (Dehydol LT 7 = C12-18 Fettalkohol, ethoxyliert (7 EO)) und 0,1 Gew.-% Phenylethanol hergestellt. Als Referenz wurde eine wässrige Lösung enthaltend 0,044 Gew.-% lineares Alkylbenzolsulfonat, 0,044 Gew.-% Dehydol LT 7 und 0,1 Gew.-% Phenylethanol hergestellt. In beide Lösungen wurde je ein 25 mm x 50 mm großer Stoffstreifen (Polyester; Flächengewicht 130 g/m²) eingetaucht, wieder herausgenommen, getrocknet und durch Abriechen von geruchlich geschulten Experten olfaktorisch beurteilt. An dem in die Referenzlösung getauchten Polyesterstreifen konnte Phenylethanol bereits nach 30 min nicht mehr wahrgenommen werden. An dem in die den erfindungsgemäßen Hydrogelbildner aus dem eben genannten Synthesebeispiel (1.) enthaltende Lösung getauchten Polyesterstreifen konnte Phenylethanol noch nach 4 h deutlich wahrgenommen werden.

### 2.3. Anwendung auf WC-Keramik

Getestet wurde eine 0,1 Gew.-%ige wässerige Lösung des erfindungsgemäßen Hydrogelbildners aus dem eben genannten Synthesebeispiel (1.) auf WC-Keramik gegen unbehandelte WC-Keramik.

Vorgehensweise:
WC-Keramiken der Firma Villeroy & Boch wurden einem üblichen Reinigungsverfahren unterzogen und über Nacht an der Luft getrocknet.
Die WC-Keramiken werden mit einer 0,1 Gew.-%igen wässerigen Lösung des erfindungsgemäßen Hydrogelbildners aus dem eben genannten Synthesebeispiel (1) mittels Triggeraufsatz gleichmäßig angesprüht und über Nacht stehend getrocknet.
Zum Testen der Abspülbarkeit wurden an definierten Stellen jeweils 0,1 ml eines artifiziellen Fäkalschmutzes auf die WC-Keramik aufgebracht, mit einer definierten Menge kaltem Leitungswasser abgespült und die Ablösung des Schmutzes wurde nach unten aufgeführter Skala benotet.

Beurteilt wurde wie folgt:
0 = keine Ablösung des Schmutzes
1 = ca. % des Schmutzes ist abgelöst
2 = ca. die Hälfte des Schmutzes ist abgelöst
3 = es sind nur noch minimale Reste des Schmutzes vorhanden
4 = der gesamte Schmutz ist abgelöst

**Mittelwert aus 14 WC-Keramiken bzw. 42 Messpunkten**

| | | | | | |
|---|---|---|---|---|---|
| Anzahl der Spülgänge | 1 | 2 | 3 | 4 | 5 |
| unbehandelte Proben | 0,5 | 1,1 | 1,1 | 2,0 | 2,3 |
| mit 0,1 Gew.-%ige wässerige Lösung des erfindungsgemäßen Hydrogelbildners gemäß Synthesebeispiel (1) behandelte Proben | 1,0 | 1,9 | 2,2 | 2,4 | 2,7 |

Bei der mit der 0,1 Gew.-%igen wässerigen Lösung des erfindungsgemäßen Hydrogelbildners aus dem eben genannten Synthesebeispiel (1) behandelten Keramik ergibt sich somit eine bessere Schmutzentfernung bei gleicher Anzahl Spülgänge.

### 3. Produktbeispiele

### Beispiel 3.1: Flüssiges Konditioniermittel

| | Gew.-% |
|---|---|
| Esterquat^{[a]} | 22,5 |
| Silikonöl | 5 |
| MgCl x 6H₂O | 0,5 |
| Parfüm | 1,6 |
| Hydrogelbildner^{[c]} | 0,5 |
| Wasser, vollentsalzt | ad 100 |

| | |
|---|---|
| ^{[a]} N-Methyl-N(2-hydroxyethyl)-N,N-(ditalgacyloxyethyl)ammonium-methosulfat ^{[c]} erfindungsgemäßer Hydrogelbildner aus dem Synthesebeispiel (1.) | |

Die Rezeptur wurde durch Aufschmelzen des Esterquats in Wasser hergestellt. Das aufgeschmolzene Esterquat wurde anschließend mit einem hochdispergierenden Gerät gerührt und die restlichen Komponenten wurden hinzugefügt. Die Parfümzugabe und Zugabe des Hydrogelbildners erfolgte bei leichter Rührung nach Abkühlung der Mischung auf unter 30°C.

### Beispiel 3.2: Konditioniersubstrat

Zur Herstellung des Konditioniersubstrates wurden Vliese aus Cellulose (Fläche: 24,5 cm x 39 cm) mit 20 g des flüssigen Konditioniermittels gemäß Beispiel 3.1 getränkt.

### Beispiel 3.3: Flüssiges Reinigungsmittel

| Rohstoff | Menge in Gew.-% |
|---|---|
| C12-18 Fettsäure, Na-Salz | 0,7 |
| C10-13 Alkylbenzolsulfonat | 6,4 |
| Natriumcitrat | 1,5 |
| Natriumcarbonat | 3,0 |
| Ethanol | 2,1 |
| Cumolsulfat, Na | 1,5 |
| C12-18 Fettalkohol + 7EO | 1,5 |
| C8-Fettalkoholsulfat, Na-Salz | 1,5 |
| Hydrogelbildner^{[c]} | 0,5 |
| Parfüm | 0,7 |
| Wasser | ad 100 |

| | |
|---|---|
| ^{[c]} erfindungsgemäßer Hydrogelbildner aus dem Synthesebeispiel (1.) | |

### Beispiel 3.4: Flüssigwaschmittel

| Rohstoff | Menge in Gew.-% |
|---|---|
| C12-14 Fettsäure | 8,8 |
| C12-18 Fettalkohol + 7EO | 24,0 |
| Alkylpolyglucosid | 2,0 |
| C12-14-2EO -sulfat | 5,0 |
| C16-18 Fettsäure | 6,8 |
| NaOH 50% | 3,0 |
| Citronensäure x 1H2O | 1,0 |
| Glycerin 99,5% | 7,5 |
| Ethanol | 1,0 |
| Silikonöl | 0,3 |
| Polyvinylpyrrolidon | 0,5 |
| HEDP-4Na | 0,5 |
| Enzym, Farbstoff, Parfum, | 0,8 |
| Hydrogelbildner^{[c]} | 0,7 |
| Wasser | ad 100 |

| | |
|---|---|
| ^{[c]} erfindungsgemäßer Hydrogelbildner aus dem Synthesebeispiel (1.) | |

### Beispiel 3.5: Festes Waschmittel

| Rohstoff | Menge in Gew.-% |
|---|---|
| Alkylbenzolsulfonat (Natriumsalz) | 12 |
| Carboxymethylcel lu lose | 1 |
| Enzyme | 1 |
| Niotensid | 3 |
| (1-Hydroxyethyliden)bisphosphonat | 1 |
| Natriumcarbonat | 25 |
| Natriumpercabonat | 12 |
| Natriumsulfat | 27 |
| Polyacrylat | 3 |
| Entschäumer | 2 |
| N,N,N',N'-Tetraacetylethylendiamin | 3 |
| Wasser | 3 |
| Parfüm | 0,15 |
| Hydrogelbildner^{[c]} | 1,0 |
| Natriumsilicat | ad 100 |
| Summe | 100 |

| | |
|---|---|
| ^{[c]} erfindungsgemäßer Hydrogelbildner aus dem Synthesebeispiel (1.1) Natriumsilicat: amorphes Natriumsilicat mit Na₂O:SiO₂ = 2,4 Polyacrylat: Polyacrylsäure, Natriumsalz; M = 4500 g/mol | |

### Beispiel 3.6: Waschmittel-Gel

| Rohstoff | Menge in Gew.-% |
|---|---|
| Alkylpolyglucosid | 2,00 |
| C12-14-Seife, Na | 8,80 |
| C16-18-Seife, Na | 6,80 |
| NaOH 50% | 3,00 |
| Citronensäurex1H₂O | 1,00 |
| Glycerin 99,5% | 7,50 |
| Ethanol | 1,00 |
| Silikonentschäumer | 0,30 |
| Borsäure | 1,00 |
| 1-Hydroxyethylendiphosphonsäure | 0,50 |
| Vinylimidazol-Vinylpyrrolidon-Copolymer | 1,67 |
| Hydrogelbildner^{[c]} | 0,8 |
| Parfüm | 1,3 |
| Wasser | ad 100 |

| | |
|---|---|
| ^{[c]} erfindungsgemäßer Hydrogelbildner aus dem Synthesebeispiel (1) | |

### Beispiel 3.7: Bügelwasser

| Rohstoff | Menge in Gew.-% |
|---|---|
| Ethanol | 2 |
| Wasserstoffperoxid | 0,01 |
| Parfüm | 0,05 |
| Hydrogelbildner^{[c]} | 0,02 |
| Wasser mit 5° dH | ad 100 Gew.-% |

| | |
|---|---|
| ^{[c]} erfindungsgemäßer Hydrogelbildner aus dem Synthesebeispiel (1) | |

## Patentansprüche

1. Wasch- oder Reinigungsmittel, **dadurch gekennzeichnet, dass** es zumindest einen Hydrogelbildner mit einem Molekulargewicht kleiner als 3500 g/Mol umfasst, wobei der Hydrogelbildner der folgenden Formel (I) genügt: 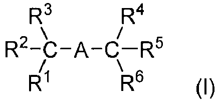 wobei den Resten A, R¹ bis R⁶ folgende Bedeutung zukommt:
A = 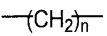 mit n = 1 bis 24, vorzugsweise 6 bis 18, insbesondere 8 bis 14,
R¹ bis R⁶, jeweils unabhängig voneinander: Alkyl-, Hydroxyalkyl, Aminoalkyl-, -COOR,
- CONRR oder 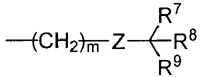 mit m= 0 - 10
darin ist Z = -CONH-(CH₂)ᵣ-, -COO-(CH₂)ᵣ-, -O-(CH₂)ᵣ- oder -CONR-(CH₂)ᵣ-,
mit r = 0 bis 10
und R⁷ bis R⁹ unabhängig voneinander = H, Alkyl, Hydroxyalkyl, Aminoalkyl, -CONRR, -COOR oder Monosaccharide,
wobei R = H, Alkyl oder Aryl ist,
wobei der Hydrogelbildner bei Konzentrationen unterhalb der minimalen Gelbildungskonzentration wasserlöslich ist,
wobei die minimale Gelbildungskonzentration diejenige Konzentration ist, bei welcher sich in einer binären Lösung aus Wasser und Hydrogelbildner bei T = 25 °C erstmals eine Fließgrenze von zumindest 0,05 Pa ausbildet,
und wobei diese minimale Gelbildungskonzentration unterhalb 2 Gew.-% Hydrogelbildner liegt,
bezogen auf das System bestehend aus Wasser und Hydrogelbildner,
und wobei das Wasch- oder Reinigungsmittel 0,1 bis 40 Gew.-% Tensid(e) umfasst.

2. Mittel gemäß Anspruch 1, **dadurch gekennzeichnet, dass** der Hydrogelbildner ein Molekulargewicht kleiner 3000 g/mol, vorzugsweise kleiner 2500 g/mol, in weiter vorteilhafter Weise kleiner 2000 g/mol, insbesondere kleiner 1500 g/mol aufweist.

3. Mittel gemäß Anspruch 1 oder 2, **dadurch gekennzeichnet, dass** der Hydrogelbildner in Mengen von 0,01 bis 20 Gew.-%, vorzugsweise von 0,1 bis 5 Gew.-%, bezogen auf das gesamte Mittel, enthalten ist.

4. Mittel gemäß einem der Ansprüche 1 bis 3, **dadurch gekennzeichnet, dass** der Hydrogelbildner der folgenden Formel (II) genügt: 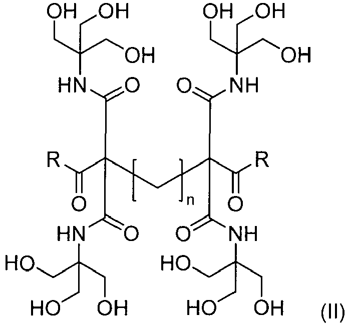
mit n = 1 bis 24, vorzugsweise 6 bis 18, insbesondere 8 bis 14,
und R = HO-, Alkyl-O- (wie insbesondere C₂H₅-O-) oder (CH₂OH)₃C-NH-.

5. Verfahren zur Erzeugung von Hydrogelschichten auf Oberflächen, insbesondere harten oder weichen Oberflächen, **dadurch gekennzeichnet, dass** die betreffende Oberfläche mit einem Wasch- oder Reinigungsmittel nach einem der Ansprüche 1 bis 4 behandelt wird oder mit einer Flüssigkeit, die den in diesen Ansprüchen genannten Hydrogelbildner umfasst.

6. Verfahren gemäß Anspruch 5, **dadurch gekennzeichnet, dass** bei der Schichtbildung Aktivstoffe, wie insbesondere Riechstoffe, Pflegestoffe, antimikrobielle Wirkstoffe in die Schichten ein- bzw. an die Schichten angelagert werden.

7. Verfahren nach einem der Ansprüche 5 oder 6 zur (a) Hydrophilierung von harten oder weichen Oberflächen, (b) Erleichterung der Entfernbarkeit von Schmutz von harten oder weichen Oberflächen, (c) Verbesserung des Schmutzabweisevermögens harter oder weicher Oberflächen, (d) retardierten Freisetzung von Wirkstoffen, insbesondere Riechstoffen und antimikrobiellen Stoffen, welche in den Gelschichten gespeichert sind, und/oder (e) Verbesserung des Weichgriffes von Textilien.

8. Verfahren gemäß einem der Ansprüche 5 bis 7, **dadurch gekennzeichnet, dass** es in einer automatischen Waschmaschine durchgeführt wird, vorzugsweise bei Temperaturen ≤ 40 °C, insbesondere ≤ 30 °C.

9. Textilwaschverfahren, **dadurch gekennzeichnet, dass** es unter Einsatz eines Wasch- mittels nach einem der Ansprüche 1 bis 4 durchgeführt wird.

10. Reinigungsverfahren für harte Oberflächen, **dadurch gekennzeichnet, dass** es unter Einsatz eines Reinigungsmittels nach einem der Ansprüche 1 bis 4 durchgeführt wird.

## Claims

1. Washing or cleaning agent, **characterised in that** it contains at least one hydrogelator with a molecular weight of less than 3500 g/mol, wherein the hydrogelator satisfies the following Formula (I): 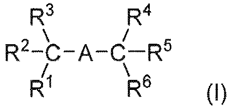 wherein the groups A, R¹ to R⁶ have the following meaning:
A = -(CH₂)ₙ- with n = 1 to 24, preferably 6 to 18, in particular 8 to 14,
R¹ to R⁶, each independently of one another: alkyl-, hydroxyalkyl-, aminoalkyl, -COOR, -CONRR or 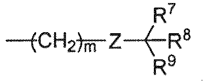 with m = 0 - 10 in which Z = -CONH-(CH₂)ᵣ-, -COO-(CH₂)ᵣ-, -O-(CH₂)ᵣ- or -CONR-(CH₂)ᵣ-, with r = 0 to 10
and R⁷ to R⁹ independently of one another = H, alkyl, hydroxyalkyl, aminoalkyl, -CONRR, -COOR
or monosaccharides,
wherein R = H, alkyl or aryl,
wherein the hydrogelator is water-soluble at concentrations below the minimum gelation concentration,
wherein the minimum gelation concentration is that concentration, at which a yield stress of at least 0.05 Pa is formed in a binary solution of water and hydrogelator at T = 25 °C,
and wherein this minimum gelation concentration is less than 2 wt % of hydrogelator, based on the system consisting of water and hydrogelator,
and wherein the washing or cleaning agent contains 0.1 to 40 wt % surfactant(s).

2. Agent according to claim 1, **characterised in that** the molecular weight of the hydrogelator is less than 3000 g/mol, preferably less than 2500 g/mol, more advantageously less than 2000 g/mol, especially less than 1500 g/mol.

3. The agent according to claim 1 or 2, **characterised in that** the hydrogelator is comprised in amounts of 0.01 to 20 wt %, preferably 0.1 to 5 wt %, based on the total agent.

4. Agent according to one of claims 1 to 3, **characterised in that** the hydrogelator satisfies the following Formula (II): 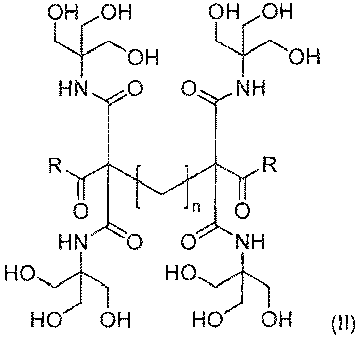
with n = 1 to 24, preferably 6 to 18, in particular 8 to 14,
and R = HO-, alkyl-O- (such as especially C₂H₅-O-) or (CH₂OH)₃C-NH-.

5. Method for the production of hydrogel layers on surfaces, especially hard or soft surfaces, **characterised in that** the surface in question is treated with a washing or cleaning agent according to one of claims 1 to 4 or with a liquid that contains the hydrogelator cited in these claims.

6. Method according to claim 5, **characterised in that** active substances, such as in particular fragrances, nurturing ingredients, antimicrobials are deposited during the layer formation into the layers in or onto the layers.

7. Method according to one of claims 5 or 6 for (a) rendering hard or soft surfaces hydrophilic, (b) facilitating the removability of soil from hard or soft surfaces, (c) improving the soil repellent capability of hard or soft surfaces, (d) retarded release of active substances, especially fragrances and antimicrobials that are sequestered in the gel layers, and/or (e) improving the soft feel of fabrics.

8. Method according to one of claims 5 to 7, **characterised in that** it is carried out in an automatic washing machine, preferably at temperatures of ≤ 40 °C, especially ≤ 30 °C.

9. Method for washing textiles, **characterised in that** it is carried out using a washing agent according to one of claims 1 to 4.

10. Method for cleaning hard surfaces, **characterised in that** it is carried out using a cleaning agent according to one of claims 1 to 4.

## Revendications

1. Agent de lavage ou de nettoyage, **caractérisé en ce qu'**il comprend au moins un agent de formation d'hydrogel avec une masse moléculaire inférieure à 3 500 g/mol,
dans lequel l'agent de formation d'hydrogel satisfait à la Formule (I) suivante : 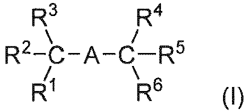
dans laquelle la signification suivante s'applique aux radicaux A, R¹ à R⁶ :
A = 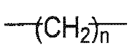 avec n = de 1 à 24, de préférence de 6 à 18, en particulier de 8 à 14,
R¹ à R⁶, respectivement indépendamment les uns des autres : alkyle-, un hydroxyalkyle, aminoalkyle-, -COOR, -CONRR ou 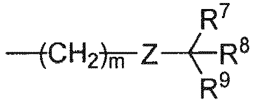 avec m = de 0 à 10
où Z = -CONH-(CH₂)ᵣ-, -COO-(CH₂)ᵣ-, -O-(CH₂)ᵣ- ou -CONR-(CH₂)ᵣ-, avec r = de 0 à 10
et R⁷ à R⁹, indépendamment les uns des autres = H, un alkyle, un hydroxyalkyle, un aminoalkyle, -CONRR, -COOR ou des monosaccharides, où R = H, un alkyle ou un aryle,
dans lequel l'agent de formation d'hydrogel est hydrosoluble à des concentrations au-dessous de la concentration de gélification minimale,
la concentration de gélification minimale étant la concentration à laquelle une limite d'écoulement d'au moins 0,05 Pa se forme pour la première fois dans une solution binaire à base d'eau et d'agent de formation d'hydrogel à T = 25 °C,
et cette concentration de gélification minimale se situant au-dessous de 2 % en poids d'agent de formation d'hydrogel, rapporté au système constitué d'eau et d'agent de formation d'hydrogel,
et l'agent de lavage ou de nettoyage contenant de 0,1 à 40 % en poids d'agent(s) tensioactif(s).

2. Agent selon la revendication 1, **caractérisé en ce que** l'agent de formation d'hydrogel présente une masse moléculaire inférieure à 3 000 g/mol, de préférence inférieure à 2 500 g/mol, de manière encore plus avantageuse inférieure à 2 000 g/mol, en particulier inférieure à 1 500 g/mol.

3. Agent selon la revendication 1 ou la revendication 2, **caractérisé en ce que** l'agent de formation d'hydrogel est contenu dans des quantités allant de 0,01 à 20 % en poids, de préférence de 0,1 à 5 % en poids, rapporté au produit total.

4. Agent selon l'une des revendications 1 à 3, **caractérisé en ce que** l'agent de formation d'hydrogel satisfait à la Formule (II) suivante : 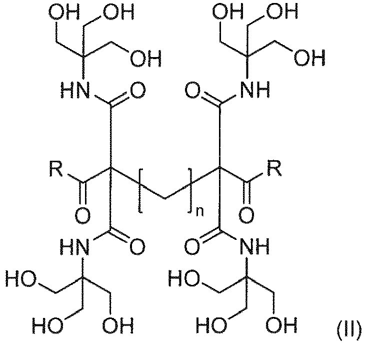
avec n = de 1 à 24, de préférence de 6 à 18, en particulier de 8 à 14,
et R = HO-, alkyle-O- (comme en particulier C₂H₅-O-) ou (CH₂OH)₃C-NH-.

5. Procédé de production de couches d'hydrogel sur des surfaces, en particulier des surfaces dures ou souples, **caractérisé en ce que** la surface concernée est traitée avec un agent de lavage ou de nettoyage selon l'une des revendications 1 à 4 ou avec un liquide qui comporte ledit agent de formation d'hydrogel indiqué dans ces revendications.

6. Procédé selon la revendication 5, **caractérisé en ce que**, lors de la formation de couches, des substances actives, comme en particulier des substances odorantes, des substances de soin, des substances actives antimicrobiennes, sont incorporées dans les couches ou déposées sur les couches.

7. Procédé selon l'une des revendications 5 ou 6 (a) d'hydrophilisation de surfaces dures ou souples, (b) de facilitation de la capacité d'enlèvement de salissures de surfaces dures ou souples, (c) d'amélioration du pouvoir de résistance aux salissures de surfaces dures ou souples, (d) de libération retardée de substances actives, en particulier de substances odorantes et de substances antimicrobiennes, lesquelles sont stockées dans les couches de gel, et/ou (e) d'amélioration du toucher de textiles.

8. Procédé selon l'une des revendications 5 à 7, **caractérisé en ce qu'**il est mis en oeuvre dans une machine à laver automatique, de préférence à des températures ≤ 40 °C, en particulier ≤ 30 °C.

9. Procédé de lavage de textiles, **caractérisé en ce qu'**il est mis en oeuvre à l'aide d'un agent de lavage selon l'une des revendications 1 à 4.

10. Procédé de nettoyage pour surfaces dures, **caractérisé en ce qu'**il est mis en oeuvre à l'aide d'un agent de nettoyage selon l'une des revendications 1 à 4.
